# EUROPÄISCHE PATENTSCHRIFT

(11) **EP 0 888 361 B1**
(45) Veröffentlichungstag und Bekanntmachung des Hinweises auf die Patenterteilung: **23.10.2002**
(21) Anmeldenummer: 97908218.7
(22) Anmeldetag: 12.03.1997
(51) Int. Cl.: C07D 521/00, A01N 43/66, A01N 43/68, A01N 43/54, A01N 43/70

(54) **DISUBSTITUIERTE METHYLIDENHYDRAZINOPHENYLSULFONYLHARNSTOFFE, VERFAHREN ZU IHRER HERSTELLUNG UND IHRE VERWENDUNG ALS HERBIZIDE UND PFLANZENWACHSTUMSREGULATOREN**
DISUBSTITUTED METHYLIDENE HYDRAZINOPHENYL SULPHONYL UREAS, PROCESS FOR THEIR PRODUCTION AND THEIR USE AS HERBICIDES AND PLANT GROWTH REGULATORS
METHYLIDENE-HYDRAZINO-PHENYLSULFONYL-UREES DISUBSTITUEES, LEUR PROCEDE DE PRODUCTION ET LEUR UTILISATION COMME HERBICIDES ET COMME REGULATEURS DE LA CROISSANCE DES PLANTES

(30) Priorität: 22.03.1996 DE 19611355
(43) Veröffentlichungstag der Anmeldung: 07.01.1999
(73) Patentinhaber: Bayer CropScience GmbH, 65929 Frankfurt/Main (DE)
(72) Erfinder: KEHNE, Heinz, D-65719 Hofheim (DE); WILLMS, Lothar, D-65719 Hofheim (DE); BAUER, Klaus, D-63456 Hanau (DE); BIERINGER, Hermann, D-65817 Eppstein (DE); ROSINGER, Christopher, D-65719 Hofheim (DE)
(86) Internationale Anmeldenummer: EP9701244
(87) Internationale Veröffentlichungsnummer: WO97035863

(56) Entgegenhaltungen:
- EP-A- 0 382 437
- EP-A- 0 562 575
- DE-A- 4 442 229
- US-A- 5 449 812

## Beschreibung

Es ist bekannt, daß Phenylsulfonylharnstoffe mit Hydrazin-Partialstrukturen herbizide Eigenschaften besitzen. Dabei handelt es sich vornehmlich um Hydrazone (EP-A-382 437, EP-A-562 575) oder um Heterocyclen mit inkorporierter Hydrazinstruktur (EP-A-382 436, EP-A-384 602).

In den deutschen Patentanmeldungen Nr. P 44 42 229.6 und Nr. 19 521 668.7 wurden bereits Phenylsulfonylharnstoffe vorgeschlagen, die in 2-Stellung Carboxy(derivat)gruppen bzw. Schwefelsubstituenten aufweisen und in 5-Stellung mit Hydrazonresten substituiert sind.

Überraschenderweise wurden nun Phenylsulfonylharnstoffe mit bestimmten, elektronenziehende Substituenten tragenden Hydrazonresten gefunden, die sich besonders gut als Herbizide oder Pflanzenwachstumsregulatoren eignen.

Gegenstand der vorliegenden Erfindung sind Verbindungen der Formel (I) oder deren Salze, 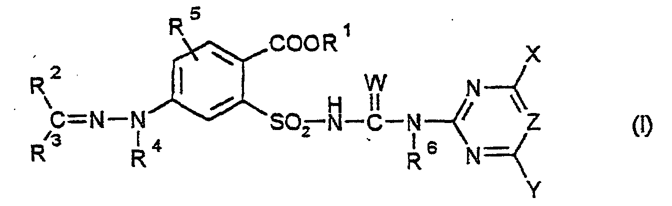 worin
- R¹: H, (C₁-C₆)Alkyl, (C₃-C₆)Alkenyl, (C₃-C₆)Alkinyl, wobei jeder der drei letztgenannten Resteunsubstituiert oder durch einen oder mehrere Reste aus der Gruppe Halogen, Phenyl, (C₁-C₄)Alkoxy, (C₁-C₄)Alkylthio und [(C₁-C₄)Alkoxy]-carbonyl substituiert ist, oder (C₃-C₆)Cycloalkyl, (C₃-C₆)Cycloalkyl(C₁-C₃)alkyl, 3-Oxetanyl, wobei jeder der letztgenannten 3 Reste unsubstituiert oder durch einen oder mehrere Reste aus der Gruppe Halogen, (C₁-C₄)Alkyl und (C₁-C₄)Alkoxy substituiert ist,
a)
   R² CN, NO₂ oder Acyl und
   R³ CN, NO₂, Acyl, CF₃, oder Aryl, welches unsubstituiert oder substituiert ist durch einen oder mehrere Reste aus der Gruppe Oxo, Halogen, Alkoxy, Haloalkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, Mono- und Dialkylamino, Acylamino, Arylamino, N-Alkyl-N-Arylamino, Alkylsulfinyl, Haloalkylsulfinyl, Alkylsulfonyl, Haloalkylsulfonyl, Alkyl oder Haloalkyl sowie den genannten gesättigten kohlenwasserstoffhaltigen Resten entsprechende ungesättigte aliphatische Reste, oder
b)
   R²R³C gemeinsam einen carbocyclischen oder heterocyclischen Ring, der in mindestens einer Nachbarstellung zu dem C-Atom in Position 1 der Gruppe CR²R³ ein C-Atom aufweist, das durch eine Oxogruppe substituiert ist, oder
c)
   R² NO₂ oder [(C₁-C₄)Alkyl]-carbonyl und
   R³ H oder (C₁-C₄)Alkyl,
- R⁴: H, einen unsubstituierten oder substituierten, aliphatischen Kohlenwasserstoffrest mit 1 bis 6 C-Atomen im.Kohlenwasserstoffteil und mit einem oder mehreren Substituenten, wobei die Substituenten aus der Gruppe Halogen, (C₁-C₄)Alkoxy, (C₁-C₄)Alkylthio, (C₁-C₄)Alkylsulfonyl, [(C₁-C₄)Alkyl]carbonyl, [(C₁-C₄)-Alkoxy]carbonyl, CN, Phenyl, das unsubstituiert oder ein- oder mehrfach substituiert ist durch gleiche oder verschiedene Reste aus der Gruppe Halogen, (C₁-C₄) Alkyl, (C₁-C₄) Alkoxy, (C₁-C₄) Halogenalkyl, (C₁-C₄) Halogenalkoxy, und Nitro, und (C₃-C₆)Cycloalkyl ausgewählt sind, oder (C₃-C₆)Alkenyl oder (C₃-C₆)Alkinyl oder einen Acylrest mit vorzugsweise 1 bis 20 C-Atomen,
- R⁵: H, Halogen, NO₂, CN, (C₁-C₄)Alkyl, (C₁-C₄)Alkoxy, [(C₁-C₄)Alkyl]carbonyl oder [(C₁-C₄)Alkoxylcarbonyl, wobei jeder der letztgenannten vier Reste unsubstituiert oder im Alkylteil durch ein oder mehrere Halogenatome substituiert ist,
- R⁶: H oder (C₁-C₄)Alkyl, vorzugsweise H oder CH₃,
- W: ein Sauerstoff- oder Schwefelatom, vorzugsweise ein Sauerstoffatom,
- X, Y: unabhängig voneinander H, Halogen, (C₁-C₄)Alkyl, (C₁-C₄)Alkoxy, (C₁-C₄)Alkylthio, wobei jeder der letztgenannten 3 Reste unsubstituiert oder durch einen oder mehrere Reste aus der Gruppe Halogen, (C₁-C₄)Alkoxy und (C₁-C₄)Alkylthio substituiert ist, oder Mono- oder Di-[(C₁-C₄)alkyl]-amino, (C₃-C₆)Cycloalkyl, (C₂-C₅)Alkenyl, (C₃-C₅)Alkinyl, (C₃-C₅)-Alkenyloxy oder (C₂-C₅)Alkinyloxy und
- Z: CH oder N
bedeuten.

Die Verbindungen der Formel (I) können Salze bilden, bei denen der Wasserstoff der -SO₂-NH-Gruppe durch ein für die Landwirtschaft geeignetes Kation ersetzt wird. Diese Salze sind beispielsweise Metallsalze, insbesondere Alkalimetallsalze oder Erdalkalimetallsalze, insbesondere Natrium- und Kaliumsalze, oder auch Ammoniumsalze oder Salze mit organischen Aminen. Ebenso kann Salzbildung durch Anlagerung einer Säure an basischen Gruppen, wie z.B. Amino und Alkylamino, erfolgen. Geeignete Säuren hierfür sind starke anorganische und organische Säuren, beispielsweise HCI, HBr, H₂SO₄ oder HNO₃.

In Formel (I) und allen nachfolgenden Formeln können die Reste Alkyl, Alkoxy, Haloalkyl, Hatoalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Alkenyl in der Form "(C₃-C₄)Alkenyl" oder "(C₃-C₆)Alkenyl" bedeutet vorzugsweise einen Alkenylrest mit 3 bis 4 bzw. 3 bis 6 C-Atomen, bei dem die Doppelbindung nicht an dem C-Atom liegt, das mit dem übrigen Molekülteil der Verbindung (I) verbunden ist ("yl"-Position). Entsprechendes gilt für (C₃-C₄)Alkinyl usw.

Cycloalkyl bedeutet ein carbocyclisches, gesättigtes Ringsystem mit 3-8 C-Atomen, z.B. Cyclopropyl, Cyclopentyl oder Cyclohexyl.

Halogen bedeutet beispielsweise Fluor, Chlor, Brom oder lod. Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Ein Kohlenwasserstoffrest ist ein geradkettiger, verzweigter oder cyclischer und gesättigter oder ungesättigter aliphatischer oder aromatischer Kohlenwasserstoffrest, z.B. Alkyl, Alkenyl, Alkinyl, Cycloalkyl, Cycloalkenyl oder Aryl; Aryl bedeutet dabei ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl; vorzugsweise bedeutet ein Kohlenwasserstoffrest Alkyl, Alkenyl oder Alkinyl mit bis zu 12 C-Atomen oder Cycloalkyl mit 3, 4, 5, 6 oder 7 Ringatomen oder Phenyl; entsprechendes gilt für einen Kohlenwasserstoffrest in einem Kohlenwasserstoffoxyrest.

Ein heterocyclischer Rest oder Ring (Heterocyclyl) kann gesättigt, ungesättigt oder heteroaromatisch sein; er enthält vorzugsweise ein oder mehrere Heteroeinheiten im Ring, vorzugsweise aus der Gruppe N, O, S, SO, SO₂; vorzugsweise ist er ein aliphatischer Heterocyclylrest mit 3 bis 7 Ringatomen oder ein heteroaromatischer Rest mit 5 oder 6 Ringatomen und enthält 1, 2 oder 3 Heteroeinheiten. Der heterocyclische Rest kann z.B. ein heteroaromatischer Rest oder Ring (Heteroaryl) sein, wie z.B. ein mono-, bioder polycyclisches aromatisches System, in dem mindestens 1 Ring ein oder mehrere Heteroatome enthält, beispielsweise Pyridyl, Pyrimidinyl, Pyridazinyl, Pyrazinyl, Thienyl, Thiazolyl, Oxazolyl, Furyl, Pyrrolyl, Pyrazolyl und Imidazolyl, oder ist ein partiell oder vollständig hydrierter Rest wie Oxiranyl, Pyrrolidyl, Piperidyl, Piperazinyl, Dioxolanyl, Morpholinyl, Tetrahydrofuryl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.
Substituierte Reste, wie substituierte Kohlenwasserstoffreste, z.B. substituiertes Alkyl, Alkenyl, Alkinyl, Aryl, Phenyl und Benzyl, oder substituiertes Heterocyclyl oder Heteroaryl, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Monound Dialkylamino, und Alkylsulfinyl, Haloalkylsulfinyl, Alkylsulfonyl, Haloalkylsulfonyl und, im Falle cyclischer Reste, auch Alkyl und Haloalkyl sowie den genannten gesättigten kohlenwasserstoffhaltigen Resten entsprechende ungesättigte aliphatische Reste, wie Alkenyl, Alkinyl, Alkenyloxy, Alkinyloxy etc. bedeuten. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy und Chlor.

Mono- oder disubstituiertes Amino bedeutet einen chemisch stabilen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Monoalkylamino, Dialkylamino, Acylamino, Arylamino, N-Alkyl-N-arylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Halogenalkyl, (C₁-C₄)Halogenalkoxy und Nitro substituiert ist, z.B. o-, m- und p-Tolyl, Dimethylphenyle, 2-, 3- und 4-Chlorphenyl, 2-, 3- und 4-Trifluor- und -Trichlorphenyl, 2,4-, 3,5-, 2,5- und 2,3-Dichlorphenyl, o-, m- und p-Methoxyphenyl.

Ein Acylrest bedeutet den Rest einer organischen Säure, z.B. den Rest einer Carbonsäure und Reste davon abgeleiteter Säuren wie der Thiocarbonsäure, gegebenenfalls N-substituierten Iminocarbonsäuren oder den Rest von Kohlensäuremonoestern, gegebenenfalls N-substituierter Carbaminsäure, Sulfonsäuren, Sulfinsäuren, Phosphonsäuren, Phosphinsäuren. Acyl bedeutet beispielsweise Formyl, Alkylcarbonyl wie (C₁-C₄-Alkyl)-carbonyl, Phenylcarbonyl, wobei der Phenylring substituiert sein kann, z.B. wie oben für Phenyl gezeigt, oder Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in der allgemeinen Formel (I) nicht gesondert angegeben sind. Die durch ihre spezifische Raumform definierten möglichen Stereoisomeren, wie Enantiomere, Diastereomere, Z- und E-lsomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Die vorstehenden Beispiele für Reste oder Restebereiche, die unter die allgemeinen Begriffe wie "Alkyl", "Acyl", "substituierten Reste" etc., fallen bedeuten keine voltständige Aufzählung. Die allgemeinen Begriffe umfassen auch die weiter unten angeführten Definitionen für Restebereiche in Gruppen bevorzugter Verbindungen, insbesondere Restebereiche, welche spezifische Reste aus den Tabellenbeispielen umfassen.

Vor allem aus Gründen der höheren herbiziden Wirkung, besseren Selektivität und/oder besseren Herstellbarkeit sind erfindungsgemäße Verbindungen der Formel (I) oder deren Salze von besonderem Interesse, worin
- R¹: H, (C₁-C₆)Alkyl, (C₃-C₆)Alkenyl, (C₃-C₆)Alkinyl, wobei jeder der drei letztgenannten Reste unsubstituiert oder durch einen oder mehrere Reste aus der Gruppe Halogen, Phenyl, (C₁-C₄)Alkoxy, (C₁-C₄)Alkylthio und [(C₁-C₄)Alkoxy]-carbonyl substituiert ist, oder (C₃-C₆)Cycloalkyl, (C₃-C₆)Cycloalkyl(C₁-C₃)alkyl, 3-Oxetenyl wobei jeder der letztgenannten 3 Reste unsubstituiert oder durch einen oder mehrere Reste aus der Gruppe Halogen, (C₁-C₄)Alkyl und (C₁-C₄)Alkoxy substituiert ist,
a)
   R² [(C₁-C₄)Alkyl]carbonyl, das unsubstituiert oder durch einen oder mehrere Reste aus der Gruppe Halogen, (C₁-C₄)Alkoxy, (C₁-C₄)Alkylthio und Phenyl substituiert ist, oder [(C₁-C₄)Alkoxy]carbonyl, das unsubstituiert oder durch einen oder mehrere Reste aus der Gruppe Halogen, (C₁-C₄)Alkoxy, Phenoxy und Phenyl substituiert ist, oder CONR⁷R⁸, CHO, CN, NO₂ oder Phenylcarbonyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Alkylthio, CN und NO₂ substituiert ist, oder (C₁-C₄)Alkylsulfonyl, (C₁-C₄)Haloalkylsulfonyl oder -P(O)[O(C₁-C₄)Alkyl]₂ und
   R³ wie unter R² definiert oder CF₃ oder Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Alkylthio, (C₁-C₄)Alkylsulfonyl, [(C₁-C₄)Alkoxy]carbonyl, CN und NO₂ substituiert ist, oder
b)
   R²R³C gemeinsam einen Ring mit 5 bis 8 Ringatomen, der carbocylisch oder heterocyclisch mit ein oder zwei Heteroatomen aus der Gruppe N, O und S ist und der durch eine oder zwei Oxogruppen jeweils in alpha-Position vom C-Atom in Position 1 der Gruppe CR²R³ substituiert und zusätzlich nicht substituiert oder zusätzlich durch einen oder mehrere Reste aus der Gruppe (C₁-C₄)Alkyl, (C₁-C₄)Haloalkyl, Halogen und Oxo substituiert ist, oder
c)
   R² NO₂ oder [(C₁-C₄)Alkyl]carbonyl und
   R³ H oder (C₁-C₄)Alkyl,
- R⁴: H oder (C₁-C₆)Alkyl, das unsubstituiert oder durch einen oder mehrere Reste aus der Gruppe Halogen, (C₁-C₄)Alkoxy, (C₁-C₄)Alkylthio, (C₁-C₄)Alkylsulfonyl, [(C₁-C₄)Alkoxy]carbonyl, CN, Phenyl und (C₃-C₆)Cycloalkyl substituiert ist, oder (C₃-C₆)Alkenyl oder (C₃-C₆)Alkinyl, wobei jeder der zwei letztgenannten Reste unsubstituiert oder durch ein oder mehrere Halogenatome substituiert ist, oder eine Gruppe der Formel 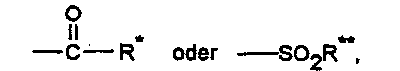 worin
R* H, (C₁-C₈)Alkyl, (C₂-C₆)Alkenyl, (C₂-C₆)Alkinyl, wobei jeder der drei letztgenannten Reste unsubstituiert oder durch einen oder mehrere Reste aus der Gruppe Halogen, (C₁-C₄)Alkoxy, (C₁-C₄)Alkylthio, Phenoxy, [(C₁-C₄)Alkoxy]carbonyl, unsubstituiertes und substituiertes Heterocyclyl und unsubstituiertes und substituiertes Phenyl substituiert ist, oder unsubstituiertes oder substituiertes (C₃-C₆)Cycloalkyl, unsubstituiertes oder substituiertes Phenyl, unsubstituiertes oder substituiertes Heterocyclyl oder [(C₁-C₄)Alkoxy]carbonyl,
R** (C₁-C₆)Alkyl, (C₃-C₆)Alkenyl, (C₃-C₆)Alkinyl, wobei jeder der drei letztgenannten Reste unsubstituiert oder durch einen oder mehrere Reste aus der Gruppe Halogen, (C₁-C₄)Alkoxy, (C₁-C₄)Alkylthio und Phenyl substituiert ist, oder Phenyl, das unsubstituiert oder durch einen oder mehrere Reste aus der Gruppe Halogen, CN, NO₂, (C₁-C₄)Alkyl, (C₁-C₄)Haloalkyl und (C₁-C₄)Alkoxy substituiert ist, bedeuten,
- R⁷ und R⁸: unabhängig voneinander H, (C₁-C₄)Alkyl, (C₃-C₄)Alkenyl, (C₃-C₄)Alkinyl oder Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Alkylthio, (C₁-C₄)Alkylsulfonyl, [(C₁-C₄)Alkoxy]carbonyl, CN und NO₂ substituiert ist, oder
- R⁷ und R⁸: gemeinsam mit dem N-Atom einen heterocyclischen Ring mit 5 oder 6 Ringgliedern, der gegebenenfalls weitere Heteroatome aus der Gruppe N, O oder S enthalten kann und unsubstituiert oder ein- oder mehrfach durch (C₁-C₄)Alkyl oder eine Oxogruppe substituiert ist,
- W: O oder S, vorzugsweise 0,
- X und Y: unabhängig voneinander H, Halogen, (C₁-C₄)Alkyl, (C₁-C₄)Alkoxy, (C₁-C₄)Alkylthio, wobei jeder der drei letztgenannten Reste unsubstituiert oder durch einen oder mehrere Reste aus der Gruppe Halogen, (C₁-C₃)Alkoxy und (C₁-C₄)Alkylthio substituiert ist, Mono- oder Di[(C₁-C₄)alkyl]amino, (C₃-C₆)-Cycloalkyl, (C₃-C₅)Alkenyl, (C₃-C₅)Alkenyloxy oder (C₃-C₅)Alkinyloxy und
- Z: CH oder N
bedeuten.

Von besonderem Interesse sind auch erfindungsgemäße Verbindungen der Formel (I) und deren Salze, worin
- R¹: (C₁-C₆)Alkyl, das unsubstituiert oder durch einen oder mehrere Reste aus der Gruppe Halogen und (C₁-C₄)Alkoxy substituiert ist, oder 3-Oxetanyl, (C₃-C₄)Alkenyl oder (C₃-C₄)Alkinyl,
a)
   R² und R³ unabhängig voneinander [(C₁-C₄)Alkyl)carbonyl, [(C₁-C₄)Alkoxy]carbonyl, CONR⁷R⁸, CHO, CN, NO₂, Benzoyl oder (C₁-C₄)Alkylsulfonyl oder
b)
   CR²R³ gemeinsam einen Ring mit 5 bis 8 Ringatomen, der carbocyclisch oder heterocyclisch mit ein oder zwei Heteroatomen aus der Gruppe N, O und S ist und der durch eine oder zwei Oxogruppen jeweils in alpha-Position zum C-Atom in Position 1 der Gruppe CR²R³ substituiert und zusätzlich nicht substituiert oder zusätzlich durch einen oder mehrere Reste aus der Gruppe (C₁-C₄)Alkyl, (C₁-C₄)Haloalkyl und Halogen substituiert ist, oder
c)
   R² NO₂ oder [(C₁-C₄)Alkyl]carbonyl und
   R³ H oder (C₁-C₄)Alkyl,
- R⁴: H, (C₁-C₄)Alkyl, das unsubstituiert oder durch einen oder mehrere Reste aus der Gruppe Halogen, (C₁-C₄)Alkoxy, (C₁-C₄)Alkylthio,
[(C₁-C₄)Alkoxy]carbonyl und Phenyl substituiert ist oder (C₃-C₄)Alkenyl oder (C₃-C₄)Alkinyl,
- R⁵: H, (C₁-C₄)Alkyl, (C₁-C₄)Haloalkyl, (C₁-C₄)Alkoxy oder Halogen,
- X und Y: unabhängig voneinander (C₁-C₄)Alkyl, (C₁-C₄)Alkoxy, wobei jeder der letztgenannten zwei Reste unsubstituiert oder durch einen oder mehrere Halogenatome substituiert ist, oder (C₁-C₄)Alkylthio, Halogen oder Mono- oder Di[(C₁-C₂)alkyl]amino und
- W: ein Sauerstoffatom
bedeuten.

Bevorzugte Verbindungen der Formel (I) oder deren Salze sind solche, worin
- R⁴: H,
- R⁵: H, (C₁-C₄)Alkyl oder Halogen,
- X: (C₁-C₂)Alkyl, (C₁-C₂)Alkoxy, (C₁-C₂)Alkylthio, (C₁-C₂)Haloalkyl oder (C₁-C₂)Haloalkoxy und
- Y: (C₁-C₂)Alkyl, (C₁-C₂)Alkoxy, Halogen, NHCH₃ oder N(CH₃)₂
bedeuten.

Besonders bevorzugte Verbindungen der Formel (I) oder deren Salze sind solche, worin
- R¹: Methyl oder Ethyl,
- R² und R³: unabhängig voneinander [(C₁-C₂)Alkoxy]carbonyl, CN oder NO₂, oder
- CR²R³: gemeinsam einen Ring mit 5 oder 6 Ringatomen, der carbocyclisch oder heterocyclisch mit ein oder zwei Heteroatomen aus der Gruppe N, O und S ist und der durch eine oder zwei Oxogruppen jeweils in alpha-Position zum C-Atom in Position 1 der Gruppe CR²R³ substituiert und zusätzlich nicht substituiert oder zusätzlich durch einen oder mehrere (C₁-C₂)Alkylreste substituiert ist, und
- R⁵: H
bedeuten.

Gegenstand der vorliegenden Erfindung sind auch Verfahren zur Herstellung der erfindungsgemäßen Verbindungen der allgemeinen Formel (I) oder deren Salze, dadurch gekennzeichnet, daß man
a) eine Verbindung der Formel (II) 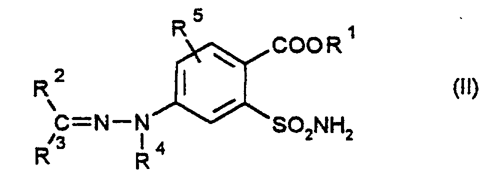 mit einem heterocyclischen Carbamat der Formel (III), 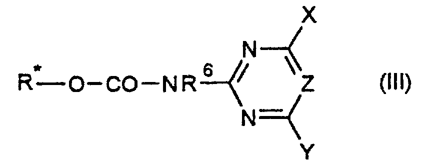 worin R* gegebenenfalls substituiertes Phenyl oder (C₁-C₄)Alkyl bedeutet, umsetzt oder
b) ein Sulfonylcarbamat der Formel (IV), 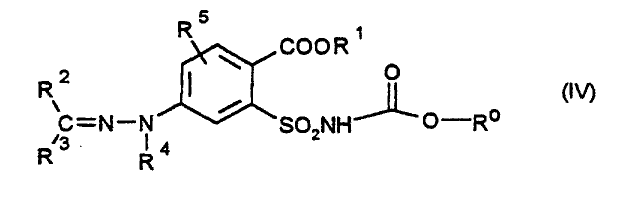 worin R^{o} gebenenfalls substituiertes Phenyl oder (C₁-C₄)Alkyl bedeutet, mit einem Aminoheterocyclus der Formel (V) 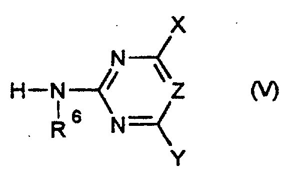 umsetzt oder
c) ein Sulfonylisocyanat der Formel (VI) 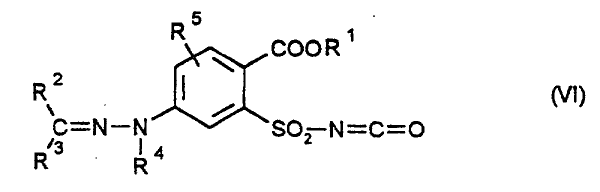 mit einem Aminoheterocyclus der Formel (V) umsetzt oder
d) ein Sulfonamid der Formel (II) mit einem (Thio)-Isocyanat der Formel (VII) 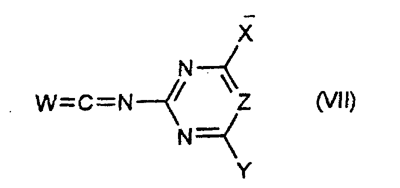 in Gegenwart einer Base umsetzt oder
e) einen Aminoheterocyclus der Formel (V) zunächst basenkatalysiert mit einem Kohlensäureester, z.B. Diphenylcarbonat, umsetzt und das gebildete Intermediat in einer Eintopfreaktion mit einem Sulfonamid der Formel (II) (siehe Variante a) umsetzt,
wobei in den Formeln (II)-(VII) die Reste bzw. Gruppen R¹-R⁶, W, X, Y und Z wie in Formel (I) definiert sind und in Verfahrensvarianten a) bis c) und e) zunächst Verbindungen (I) mit W = O erhalten werden.

Die Umsetzung der Verbindungen der Formeln (II) und (III) erfolgt vorzugsweise basenkatalysiert in einem inerten organischen Lösungsmittel, wie z.B. Dichlormethan, Acetonitril, Dioxan oder THF bei Temperaturen zwischen 0°C, vorzugsweise 20°C, und dem Siedepunkt des Lösungsmittels. Als Base werden dabei beispielsweise organische Aminbasen, wie 1,8-Diazabicyclo[5.4.0]undec-7-en (DBU) oder Alkalimetallhydroxide, wie z.B. NaOH, insbesondere bei R° = (subst.) Phenyl (vgl. EP-A-44807), oder Trimethylaluminium oder Triethylaluminium, letztere insbesondere bei R^{o} = Alkyl (vgl. EP-A-166 516) verwendet. Die jeweilige Base wird dabei beispielsweise im Bereich von 1 bis 3 Moläquivalenten, bezogen auf die Verbindung der Formel (II) eingesetzt. Die Sulfonamide (II) sind neue Verbindungen. Sie und ihre Herstellung sind ebenfalls Gegenstand dieser Erfindung.

Man erhält die Verbindungen der Formel (II) z.B. ausgehend von Verbindungen der Formel (VIII), 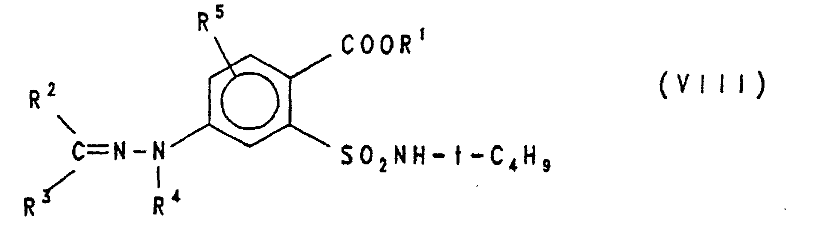 worin R¹-R⁵ wie in Formel (I) definiert sind, durch Umsetzung mit einer starken Säure (vgl. hierzu WO 89/10921).

Als starke Säuren kommen z.B. Mineralsäuren, wie H₂SO₄ oder HCl, oder starke organische Säuren, wie Trifluoressigsäure in Frage. Die Abspaltung der tert.-Butyl-Schutzgruppe erfolgt beispielsweise bei Temperaturen von -20°C bis zur jeweiligen Rückflußtemperatur des Reaktionsgemisches, vorzugsweise bei 0°C bis 40°C. Die Umsetzung kann in Substanz oder auch in einem inerten Solvens, wie z.B. Dichlormethan oder Trichlormethan, durchgeführt werden.

Die Verbindungen der Formel (VIII) erhält man z.B. aus geeigneten Aminvorstufen der Formel (IX) durch Diazotierung und anschließende Kupplung der resultierenden Diazoniumsalze mit CH-aciden Verbindungen der Formel (X) (vgl. hierzu: Houben-Weyl, "Methoden der organischen Chemie", 4. Aufl., Bd. 10/3, S. 490 ff), 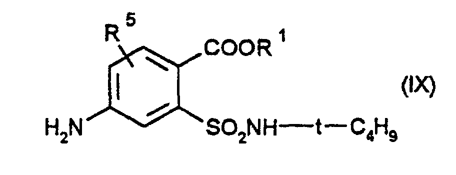 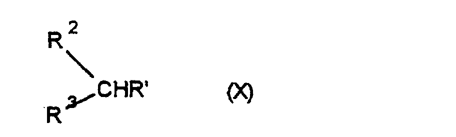 worin R' H, CO₂H, COCH₃, CO₂Me oder CO₂Et bedeutet.

In einigen Fällen ist es möglich, nach literaturbekannten Methoden im Anschluß an die Kupplung mit der CH-aciden Verbindung weitere Derivatisierungen zur Einführung von Resten R⁴ ≠ H vorzunehmen, z.B. Alkylierungen oder Acylierungen (vgl. Houben-Weyl, "Methoden der organischen Chemie", 4. Aufl. Bd. 10/2, S. 402 ff und S. 385).

Die genannten Anilin-Derivate der Formel (IX) werden nach literaturbekannten Verfahren durch Reduktion der entsprechenden Nitroverbindungen (XI) (siehe unten), z.B. durch Hydrierung mit Wasserstoff in Gegenwart eines geeigneten Katalysators, wie Pd-C oder Raney-Nickel, oder durch Reduktion mit Eisen in essigsaurem Medium erhalten; vgl. hierzu: H. Berrie, G. T. Neuhold, F. S. Spring, J. Chem. Soc. (1952), 2042; M. Freifelder, "Catalytic Hydrogenation in Organic Synthesis: Procedures and Commentary", J. Wiley and Sons, New York (1978), Kap. 5.

Die aromatischen Sulfonamide der Formel (XI) können aus den Sulfonsäuren der Formel (XII) gewonnen werden.

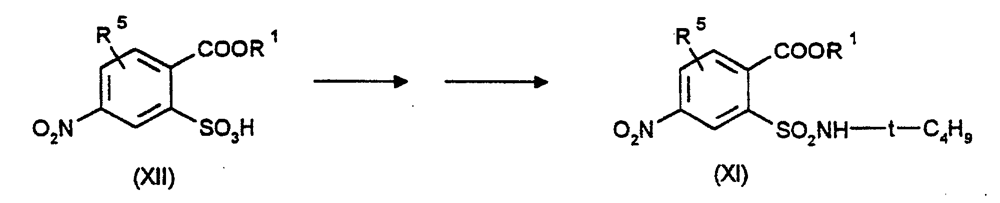

Zunächst erfolgt die Überführung der Sulfonsäuregruppe der Verbindungen (XII) in die Sulfochloride, z.B. nach Standardmethoden wie Umsetzung von Phosphoroxychlorid oder Thionylchlorid mit Kalium-Salzen der entsprechenden Sulfonsäuren in inerten Solventien wie Acetonitril und/oder Sulfolan oder in Substanz durch Erwärmen zum Rückfluß (vgl. Houben-Weyl-Klamann, "Methoden der organischen Chemie", 4. Aufl. Bd. E X1/2. S. 1067-1073, Thieme Verlag Stuttgart, 1985).

Die Sulfonamidbildung aus den Sulfochloriden mit tert.-Butylamin in Ethanol oder THF liefert die Verbindungen (XI) in guten Ausbeuten (vgl. analoge Umsetzungen in WO 89/10921).

Die Sulfonsäuren der Formel (XII) sind aus der kommerziell erhältlichen 2-Methyl-5-nitrobenzolsulfonsäure darstellbar.
Durch Oxidation der Methylgruppe von 2-Methyl-5-nitrobenzolsulfonsäure durch Standardmethoden, wie z.B. die Umsetzung mit Kaliumpermanganat zur Carbonsäurefunktion sowie nachfolgende Veresterung wird der Substituent COOR¹ eingeführt, (vgl. hierzu: Houben-Weyl-Falbe: "Methoden der organischen Chemie", 4. Aufl. Bd. E V/1, Thieme Verlag Stuttgart, 1985, S. 199-202).

Alternativ hierzu sind die Zwischenprodukte der Formel (XI) auch ausgehend von der kommerziell verfügbaren 2-Amino-5-nitrobenzoesäure nach folgendem Reaktionsschema zugänglich, 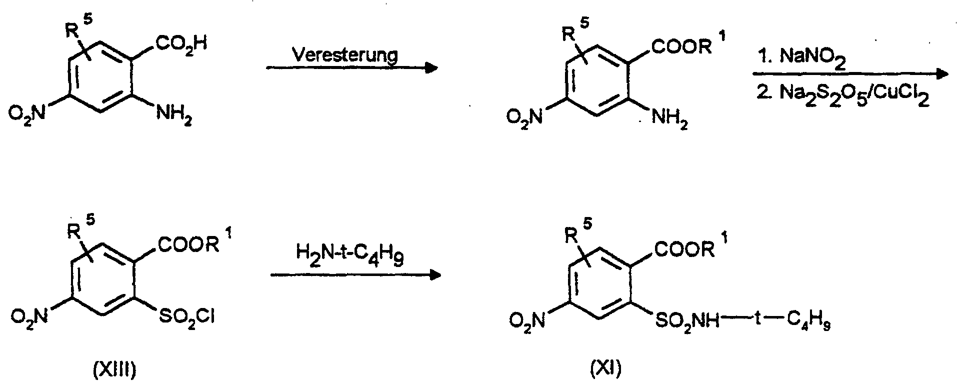 wobei alle Reaktionsschritte analog zu literaturbekannten Methoden durchgeführt werden können.

Die Verbindungen der Formel (II) lassen sich in analoger Weise auch unter Umgehung der t-Butyl-Schutzgruppe herstellen (vgl. EP 562 575). Dazu setzt man eine Verbindung der Formel (XIII) statt mit t-Butylamin mit Ammoniak um und verfährt weiter wie für Verbindung (XI) bzw. in EP 562 575 beschrieben.

Die Carbamate der Formel (III) können nach Methoden hergestellt werden, die in den südafrikanischen Patentanmeldungen 82/5671 und 82/5045 bzw. EP-A 70804 (US-A-4 480 101) oder RD 275056 beschrieben sind.

Die Umsetzung der Verbindungen (IV) mit den Aminoheterocyclen (V) führt man vorzugsweise in inerten, aprotischen Lösungsmitteln wie z.B. Dioxan, Acetonitril oder Tetrahydrofuran bei Temperaturen zwischen 0°C und der Siedetemperatur des Lösungsmittels durch. Die benötigten Ausgangsmaterialien (V) sind literaturbekannt oder können nach literaturbekannten Verfahren hergestellt werden. Die Phenylsulfonylcarbamate der Formel (IV) erhält man analog US-A-4 684 393 oder US-A-4 743 290.

Die Phenylsulfonylisocyanate der Formel (VI) lassen sich analog US-A-4 481 029 herstellen und mit den Aminoheterocyclen (V) umsetzen.

Die (Thio-)Isocyanate der Formel (VII) sind nach literaturbekannten Verfahren erhältlich (EP-A-232067, EP-A-166516). Die Umsetzung der (Thio)-lsocyanate (VII) mit Verbindungen (II) erfolgt bei -10°C bis 100°C, vorzugsweise 20 bis 100°C, in einem inerten aprotischen Lösungsmittel, wie z.B. Aceton oder Acetonitril, in Gegenwart einer geeigneten Base, z.B. N(C₂H₅)₃ oder K₂CO₃.

Die Umsetzung eines Aminoheterocyclus der Formel (V) mit Diphenylcarbonat und einem Sulfonamid der Formel (II) in einer Eintopfreaktion kann gemäß EP-A-562 575 durchgeführt werden.

Die Salze der Verbindungen der Formel (I) werden vorzugsweise in inerten polaren Lösungsmitteln wie z.B. Wasser, Methanol oder Aceton bei Temperaturen von 0-100°C hergestellt. Geeignete Basen zur Herstellung der erfindungsgemäßen Salze sind beispielsweise Alkalicarbonate, wie Kaliumcarbonat, Alkali- und Erdalkalihydroxide, z.B. NaOH oder KOH, oder Ammoniak oder Ethanolamin.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Die erfindungsgemäßen Verbindungen der Formel (I) und deren Salze, im folgenden zusammen als (erfindungsgemäße) Verbindungen der Formel (1) bezeichnet, weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.
Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.
Bei dikotylen Unkrautarten erstreckt sich das Wirkungsspektrum auf Arten wie z.B. Galium, Viola, Veronica, Lamium, Stellaria, Amaranthus, Sinapis, lpomoea, Matricaria, Abutilon und Sida auf der annuellen Seite sowie Convolvulus, Cirsium, Rumex und Artemisia bei den perennierenden Unkräutern.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen monound dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC). Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B.

Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I).
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Wirkstoffe in Mischungsformulierungen oder im Tank-Mix sind beispielsweise bekannte Wirkstoffe einsetzbar, wie sie in z.B. aus Weed Research 26, 441-445 (1986), oder "The Pesticide Manual", 10th edition, The British Crop Protection Council and the Royal Soc. of Chemistry, 1994 und dort zitierter Literatur beschrieben sind. Als literaturbekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim; ametryn; amidosulfuron; amitrol; AMS, d.h. Ammoniumsulfamat; anilofos; asulam; atrazin; azimsulfurone (DPX-A8947); aziprotryn; barban; BAS 516 H, d.h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; cafenstrole (CH-900); carbetamide; cafentrazone (ICI-A0051); CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlormesulon (ICI-A0051); chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clodinafop und dessen Esterderivate (z.B. clodinafop-propargyl); clomazone; clomeprop; cloproxydim; ctopyralid; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenil; dichlorprop; diclofop und dessen Ester wie diclofop-methyl; diethatyl; difenoxuron; difenzoquat; diflufenican; dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1H-pyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4,5-dihydro-5-oxo-1H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fiuazifop-P-butyl; fluchloralin; flumetsulam; flumeturon; flumiclorac und dessen Ester (z.B. Pentylester, S-23031); ftumioxazin (S-482); flumipropyn; flupoxam (KNW-739); fluorodifen; fluoroglycofen-ethyl; flupropacil (UBIC-4243); fluridone; flurochloridone; fluroxypyr; flurtamone; fomesafen; fosamine; furyloxyfen; glufosinate; glyphosate; halosafen; halosulfuron und dessen Ester (z.B. Methylester, NC-319); haloxyfop und dessen Ester; haloxyfop-P (= R-haloxyfop) und dessen Ester; hexazinone; imazamethabenz-methyl; imazapyr; imazaquin und Salze wie das Ammoniumsalz; imazethamethapyr; imazethapyr; imazosulfuron; ioxynil; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor; methabenzthiazuron; metham; methazole; methoxyphenone; methyldymron; metabenzuron, methobenzuron; metobromuron; metolachlor; metosulam (XRD 511); metoxuron; metribuzin; metsulfuron-methyl; MH; molinate; monalide; monocarbamide dihydrogensulfate; monolinuron; monuron; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxyfluorfen; paraquat; pebulate; pendimethalin; perfluidone; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Ester; propazine; propham; propisochlor; propyzamide; prosulfalin; prosulfocarb; prosulfuron (CGA-152005); prynachlor; pyrazolinate; pyrazon; pyrazosulfuron-ethyl; pyrazoxyfen; pyridate; pyrithiobac (KIH-2031); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy]-propansäure und -methylester; sulfentrazon (FMC-97285, F-6285); sulfazuron; sulfometuron-methyl; sulfosate (ICI-A0224); TCA; tebutam (GCP-5544); tebuthiuron; terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850); thiazafluron; thiazopyr (Mon-13200); thidiazimin (SN-24085); thifensulfuron-methyl; thiobencarb; tiocarbazil; tralkoxydim; tri-allate; triasulfuron; triazofenamide; tribenuron-methyl; triclopyr; tridiphane; trietazine; trifluralin; triflusulfuron und Ester (z.B. Methylester, DPX-66037); trimeturon; tsitodef; vernolate; WL 110547, d.h. 5-Phenoxy-1-[3-(trifluormethyl)-phenyl]-1H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

### A. Chemische Beispiele

### Beispiel A1

### 4-Amino-2-(N-tert-butylsulfamoyl)benzoesäuremethylester

Zu einem Gemisch aus 180 ml Essigsäure und 75 ml Wasser gibt man 50,0 g (0,158 mol) 2-(N-tert-Butylsulfamoyl)-4-nitrobenzoesäuremethylester (hergestellt nach DE-OS 4 236 902) und erwärmt auf 80°C. Man setzt 26,5 g (0,474 mol) Eisenpulver portionsweise so zu, daß die Temperatur nicht über 85°C ansteigt. Anschließend rührt man 4 h bei 80°C, fügt bei dieser Temperatur 85 ml 2N HCI zu und läßt auf 25°C abkühlen. Man filtriert ab und wäscht den Feststoff gründlich mit Wasser. Der Feststoff wird anschließend 3x mit je 250 ml Essigester heiß extrahiert. Die Essigesterphase dampft man ein, verreibt den Rückstand mit Diisopropylether und trocknet. Man erhält 37,7 g (83% d.Th.) 4-Amino-2-(N-tert-butylsulfamoyl)benzoesäuremethylester vom Schmp. 198-199°C.

### Beispiel A2

### 2-(N-tert.-Butylsulfamoyl)-4-(ethoxycarbonyl-nitromethylenhydrazino)-benzoesäuremethylester

5,7 g (0,02 mol) 4-Amino-2-(N-tert.-butylsulfamoyl)benzoesäuremethylester werden in einem Gemisch aus 30 ml konz. HCI und 36 ml Wasser suspendiert. Bei 0-5°C tropft man eine Lösung von 1,5 g (0,022 mol) Natriumnitrit in 9 ml Wasser zu. Man filtriert kalt von wenig ungelösten Bestandteilen ab und läßt die noch kalte Diazoniumsalzlösung bei 5-10°C in eine Mischung aus 8,9 g (0,11 mol) Natriumacetat, 16 ml Wasser, 60 ml Ethanol und 2,7 g (0,02 mol) Nitroessigsäureethylester langsam einfließen. Man rührt 2 h bei 0°C nach und läßt 15 h bei dieser Temperatur stehen. Nach Abfiltrieren, Waschen des Feststoffs mit Wasser und Trocknen im Vakuum erhält man 5,2 g (60 % d.Th.) 2-(N-tert.-Butylsulfamoyl)-4-(ethoxycarbonyl-nitromethylenhydrazino)benzoesäuremethylester vom Schmp. 142-145°C.

### Beispiel A3

### 2-(N-tert.-Butylsulfamoyl)-4-(cyano-ethoxycarbonylmethylenhydrazino)benzoesäuremethylester

4,3 g (15 mmol) 4-Amino-2-(N-tert.-butylsulfamoyl)benzoesäuremethylester werden in einem Gemisch aus 22 ml konz. HCI und 27 ml Wasser suspendiert. Bei 0-5°C tropft man eine Lösung von 1,1 g (16,5 mmol) Natriumnitrit in 7 ml Wasser zu. Nach 10 min filtriert man kalt von wenig ungelösten Bestandteilen ab und tropft die noch kalte Diazoniumsalzlösung bei 5-10°C in eine Mischung aus 6,7 g (81 mmol) Natriumacetat, 12 ml Wasser, 45 ml Ethanol und 1,7 g (15 mmol) Cyanessigsäureethylester. Man rührt 2 h bei 0°C nach und läßt 15 h bei dieser Temperatur stehen. Nach Abfiltrieren, Waschen des Feststoffs mit Wasser und Trocknen im Vakuum erhält man 2,0 g (33 % d.Th.) 2-(N-tert.-Butylsulfamoyl)-4-(cyano-ethoxycarbonylmethylenhydrazino)benzoesäuremethylester vom Schmp. 162°C.

### Beispiel A4

### 4-(Cyano-ethoxycarbonylmethylenhydrazino)-2-sulfamoylbenzoesäuremethylester

2,5 g (6 mmol) 2-(N-tert.-Butylsulfamoyl)-4-(cyano-ethoxycarbonylmethylidenhydrazino)benzoesäuremethylester werden in 30 ml Trifluoressigsäure 1,5 h bei 0-5°C gerührt. Man dampft ein und verreibt den Rückstand mit Diethylether. Nach Absaugen und Trocknen erhält man 2,0 g (92 % d.Th.) 4-(Cyanoethoxycarbonylmethylenhydrazino)-2-sulfamoylbenzoesäuremethylester vom Schmp. 188-189°C.

### Beispiel A5

### 4-(Cyano-ethoxycarbonylmethylidenhydrazino)-2-[3-(4, 6-dimethoxypyrimidin-2-yl)ureidosulfonyl]benzoesäuremethylester-Natriumsalz

Zu einer Mischung aus 1,0 g (2,8 mmol) 4-(Cyano-ethoxycarbonylmethylidenhydrazino)-2-sulfamoylbenzoesäuremethylester, 0,9 g (3,4 mmol) Phenyl-N-(4,6-dimethoxypyrimidin-2-yl)carbamat und 22 ml Acetonitril gibt man bei 0°C 0.14 g (3,5 mmol) gepulvertes Natriumhydroxid. Man rührt 2 h bei 0°C und filtriert ab. Der Feststoff wird mit wenig Diethylether gewaschen und getrocknet. Man erhält 1,3 g (83 % d.Th.) 4-(Cyano-ethoxycarbonylmethylenhydrazino)-2-[3-(4,6-dimethoxypyrimidin-2-yl)ureidosulfonyl]benzoesäuremethylester als Natriumsalz vom Schmp. 256-258°C (Zers.).

### Beispiel A6

### 4-(Cyano-ethoxycarbonylmethylidenhydrazino)-2-[3-(4,6-dimethoxypyrimidin-2-yl)-ureidosulfonyl]benzoesäuremethylester

0,7 g (1,25 mmol) 4-(Cyano-ethoxycarbonylmethylhydrazino)-2-[3-(4,6-dimethoxypyrimidin-2-yl)ureidosulfonyl]benzoesäuremethylester-Natriumsalz werden in 15 ml Wasser gelöst. Man filtriert von wenig ungelösten Bestandteilen ab und säuert das Filtrat mit 2N Salzsäure auf pH 1 an. Der ausgefallene Feststoff wird abgesaugt, mit Wasser gewaschen und getrocknet. Man erhält 0,5 g (75 % d.Th.) 4-(Cyano-ethoxycarbonylmethylidenhydrazino)-2-[3-(4,6-dimethoxypyrimidin-2-yl)ureidosulfonyl]benzoesäuremethylester vom Schmp. 145°C (Zers.)

Die in der nachfolgenden Tabelle 1 beschriebenen Verbindungen werden nach bzw. analog den obigen Beispielen A1 bis A6 erhalten.

Abkürzungen in der Tabelle:

| | |
|---|---|
| Smp. = | Fp. = Festpunkt in °C = Schmelzpunkt in °C |
| (Z) = | Schmelzpunkt unter Zersetzung |
| Ac = | Acetyl |
| Bu = | ⁿBu = n-Butyl |
| Et = | Ethyl |
| Me = | Methyl |
| Ph = | Phenyl |
| Pr = | ⁿPr = n-Propyl |
| i-Pr = | Isopropyl |
| Het = | Heterocyclus, wobei Het für einen der Reste T1 bis T15 steht |

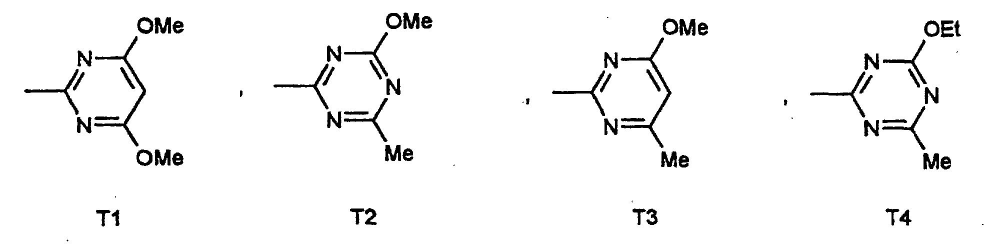

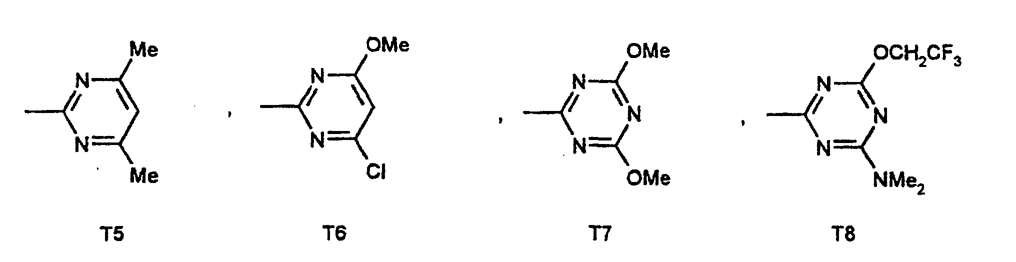

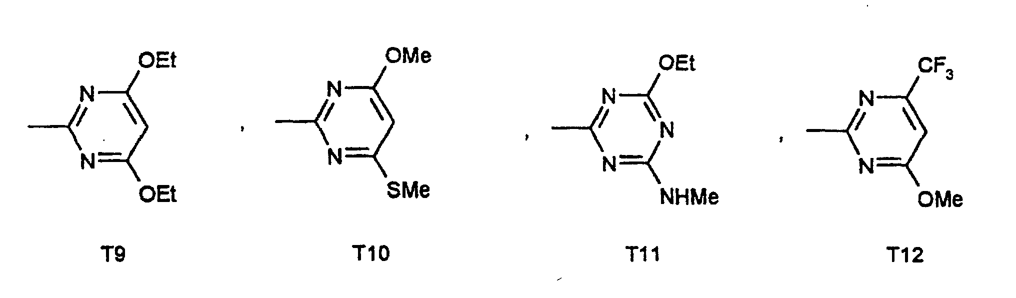

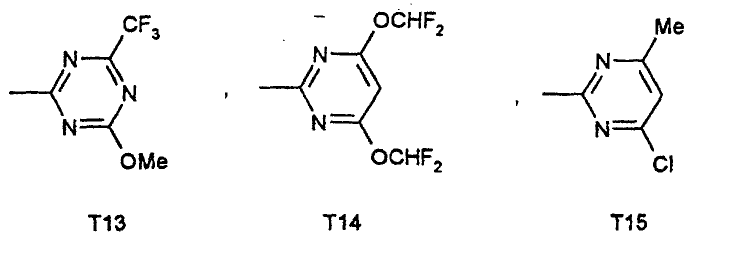

Ein Diradikal wie 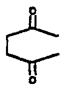 in Spalten für R², R³ heißt, daß R² und R³ zusammen die Diradikalbrücke bedeuten und mit dem C-Atom der Gruppe R²R³C = einen Alkylidenrest bilden. Entsprechendes gilt für andere Eintragungen, die für zwei Spalten gelten.

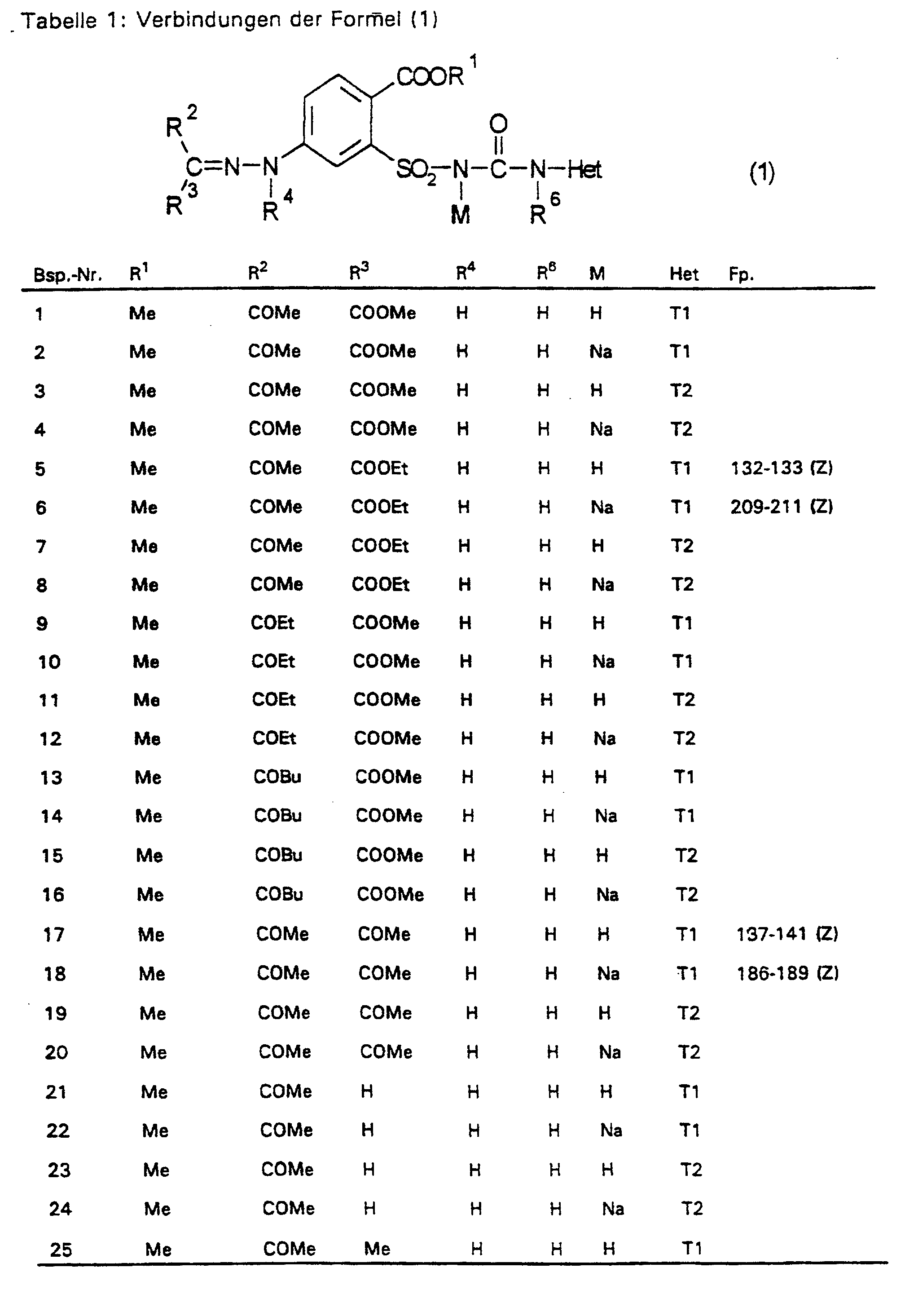

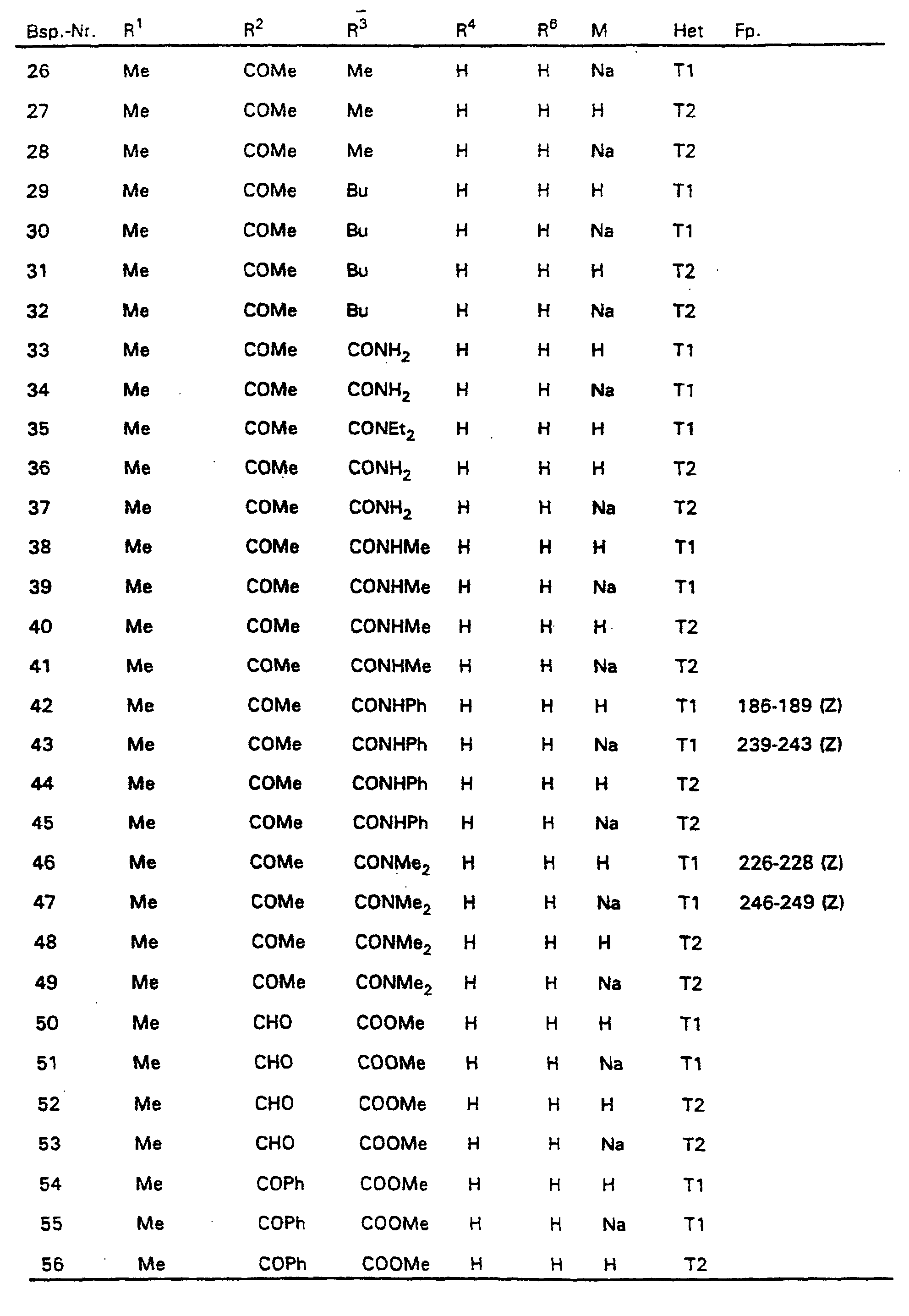

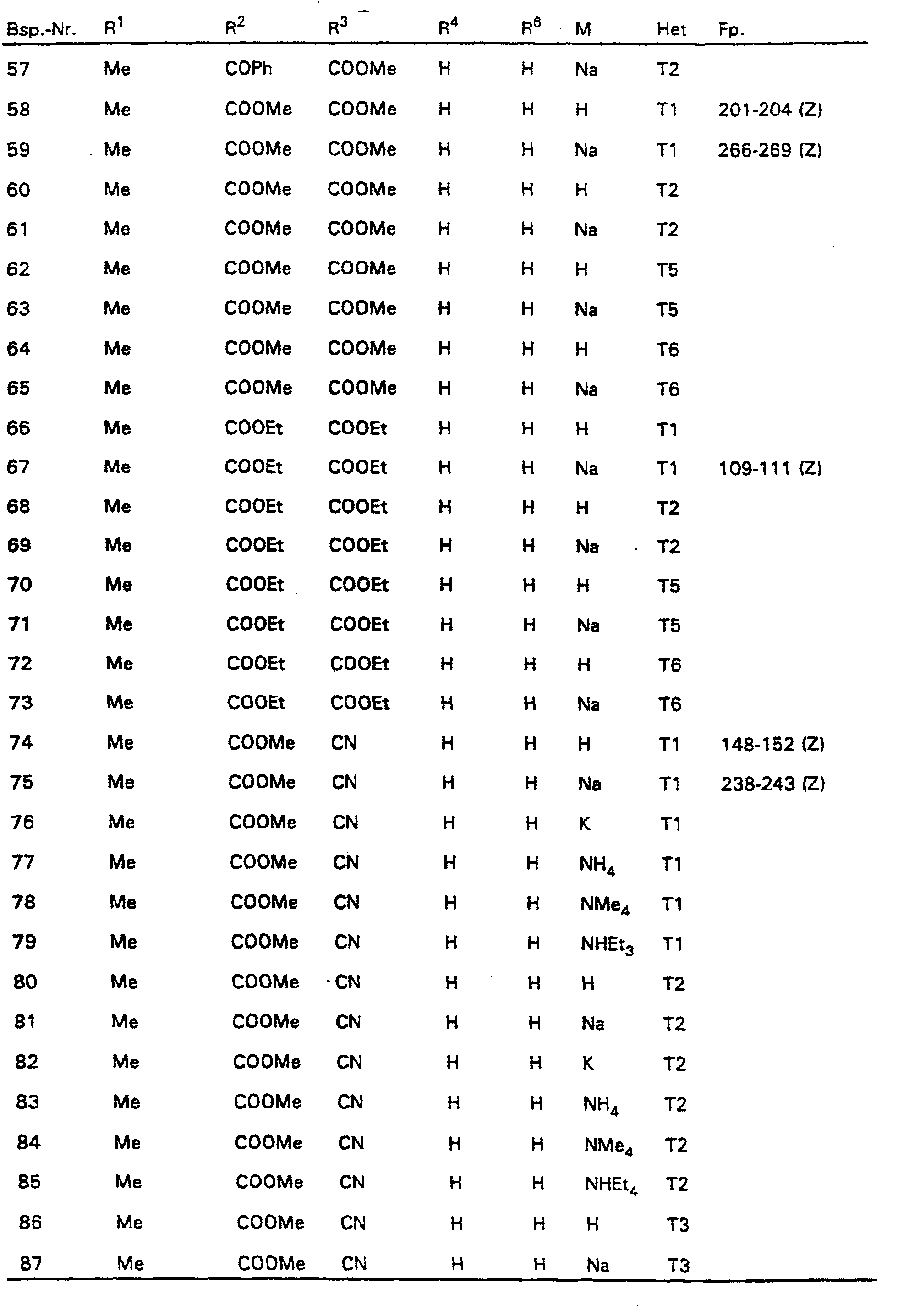

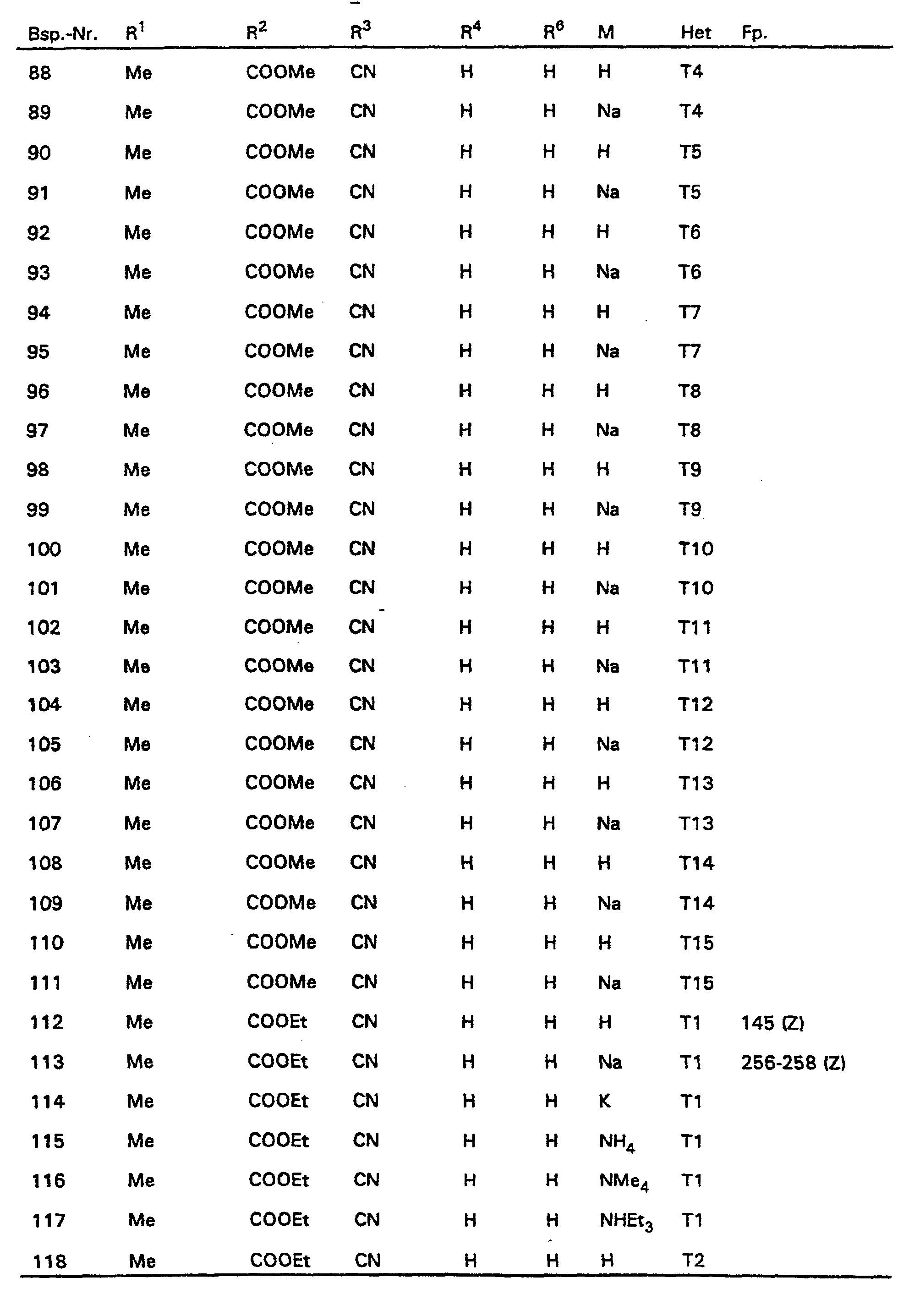

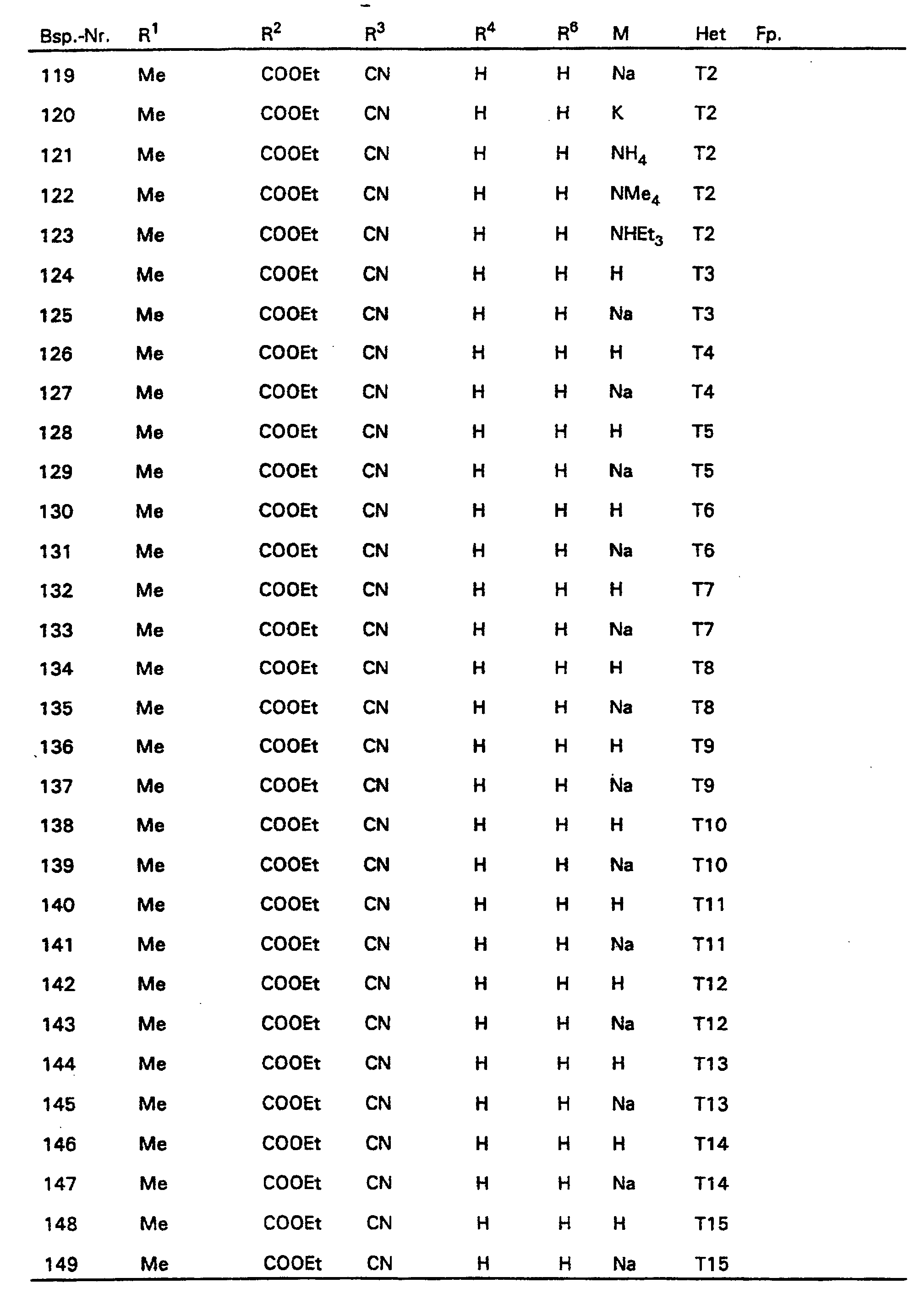

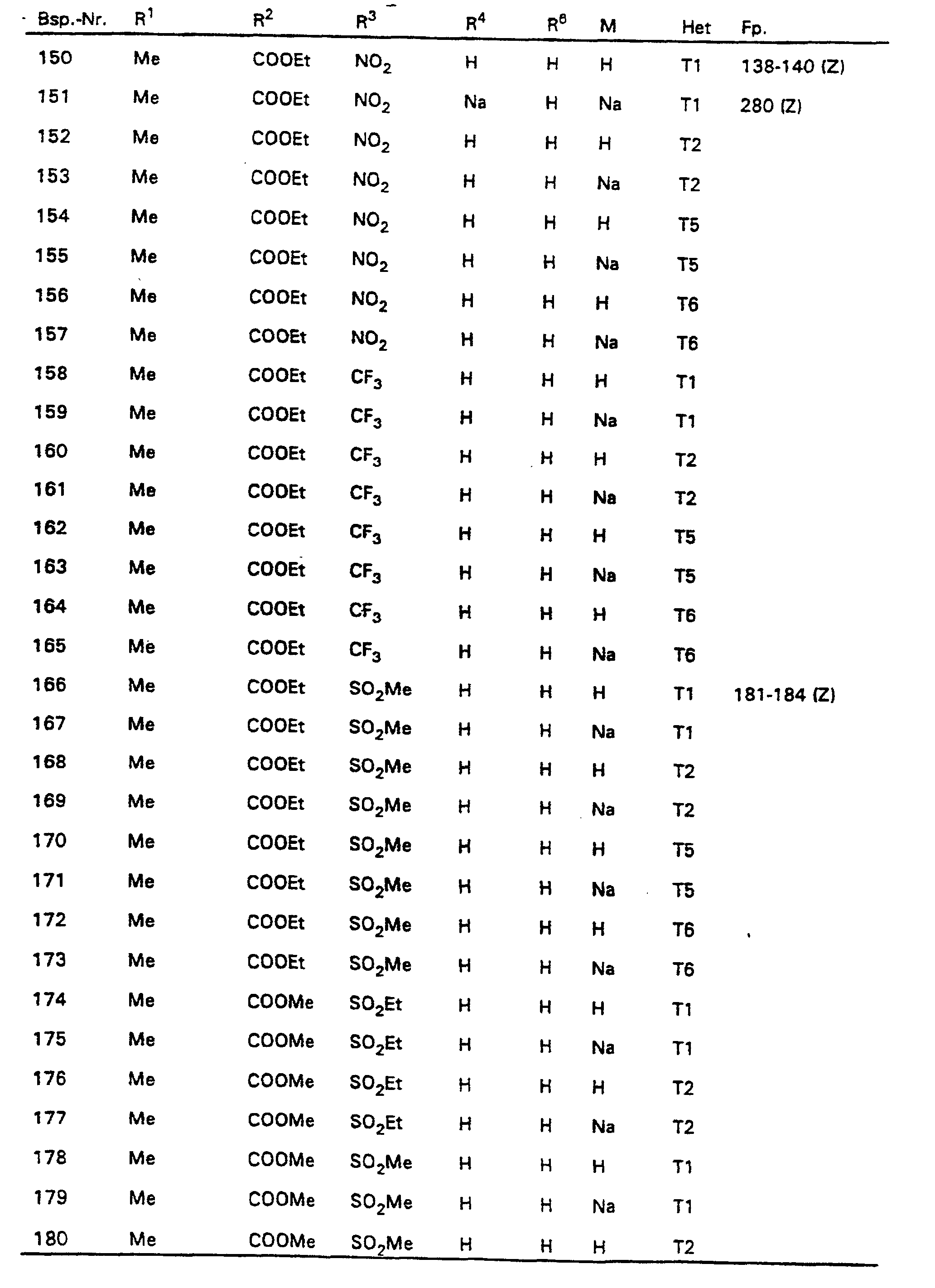

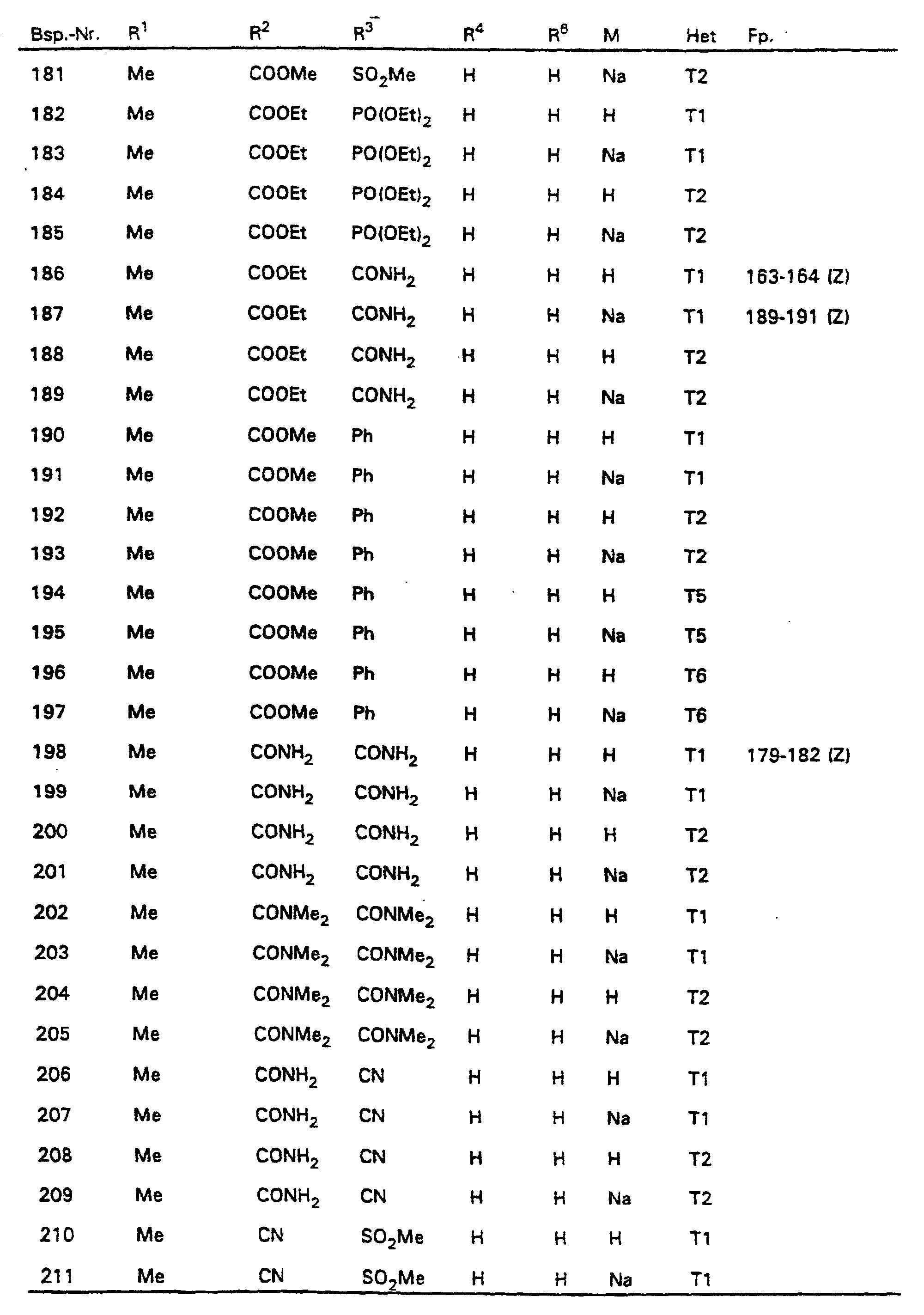

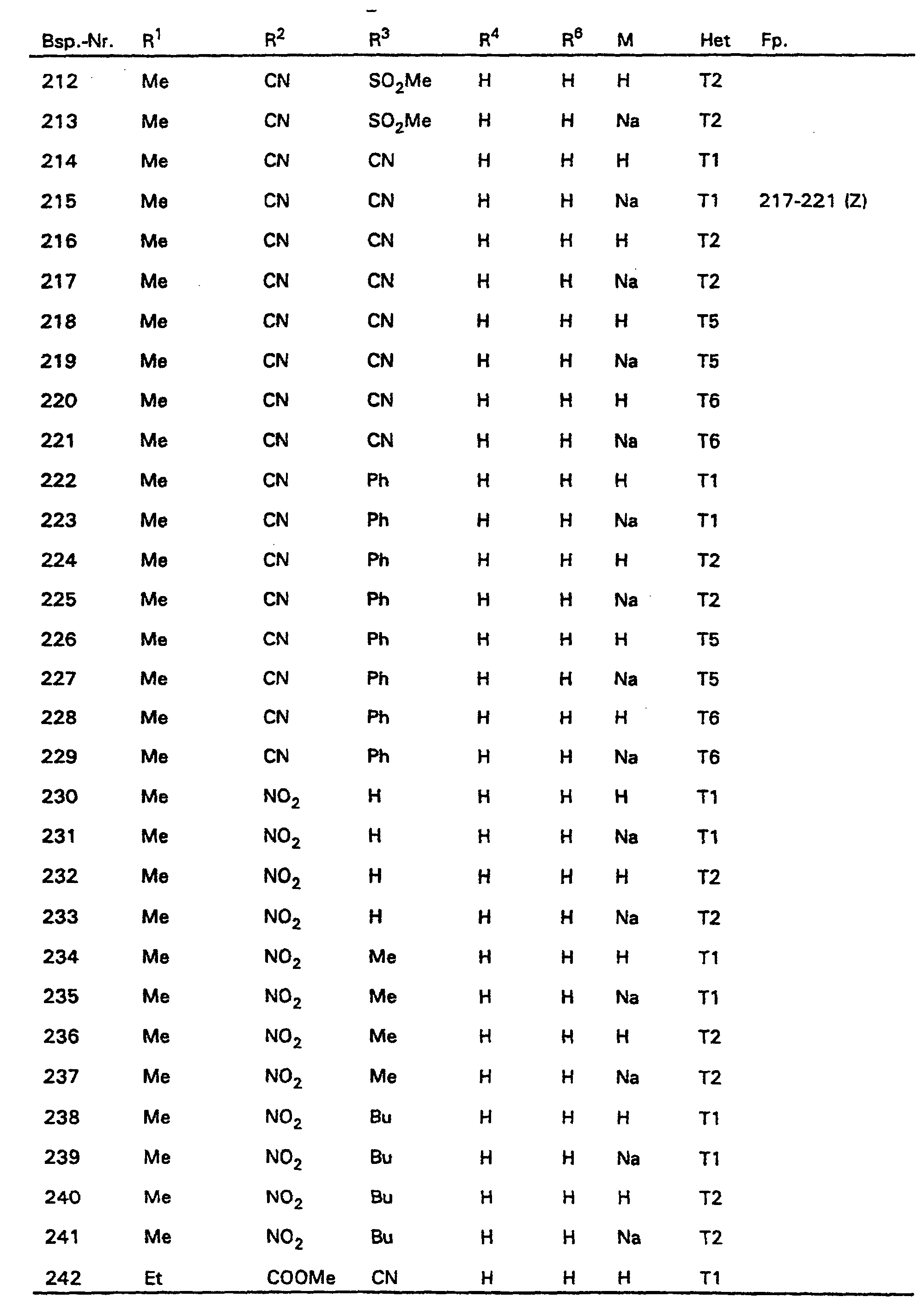

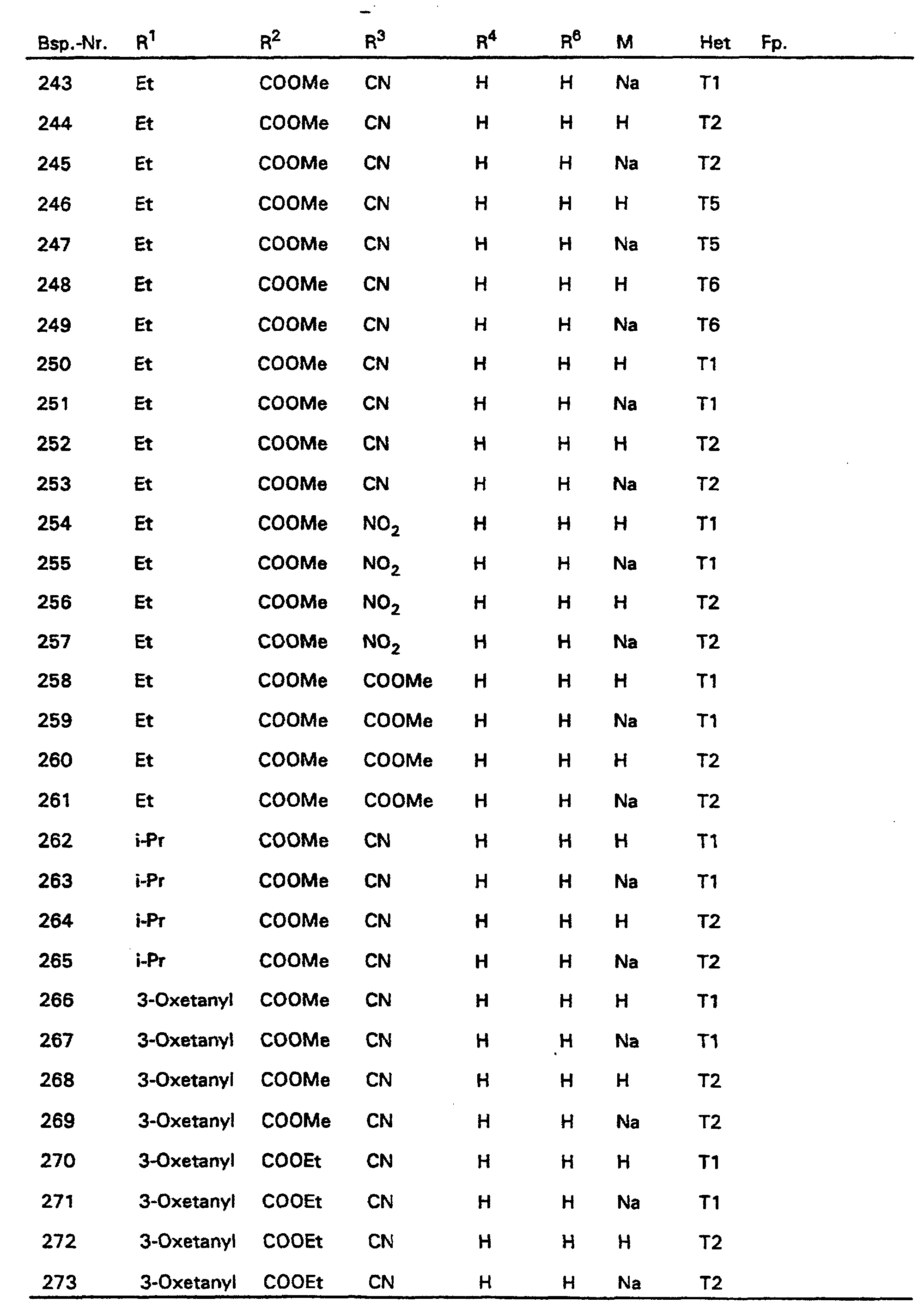

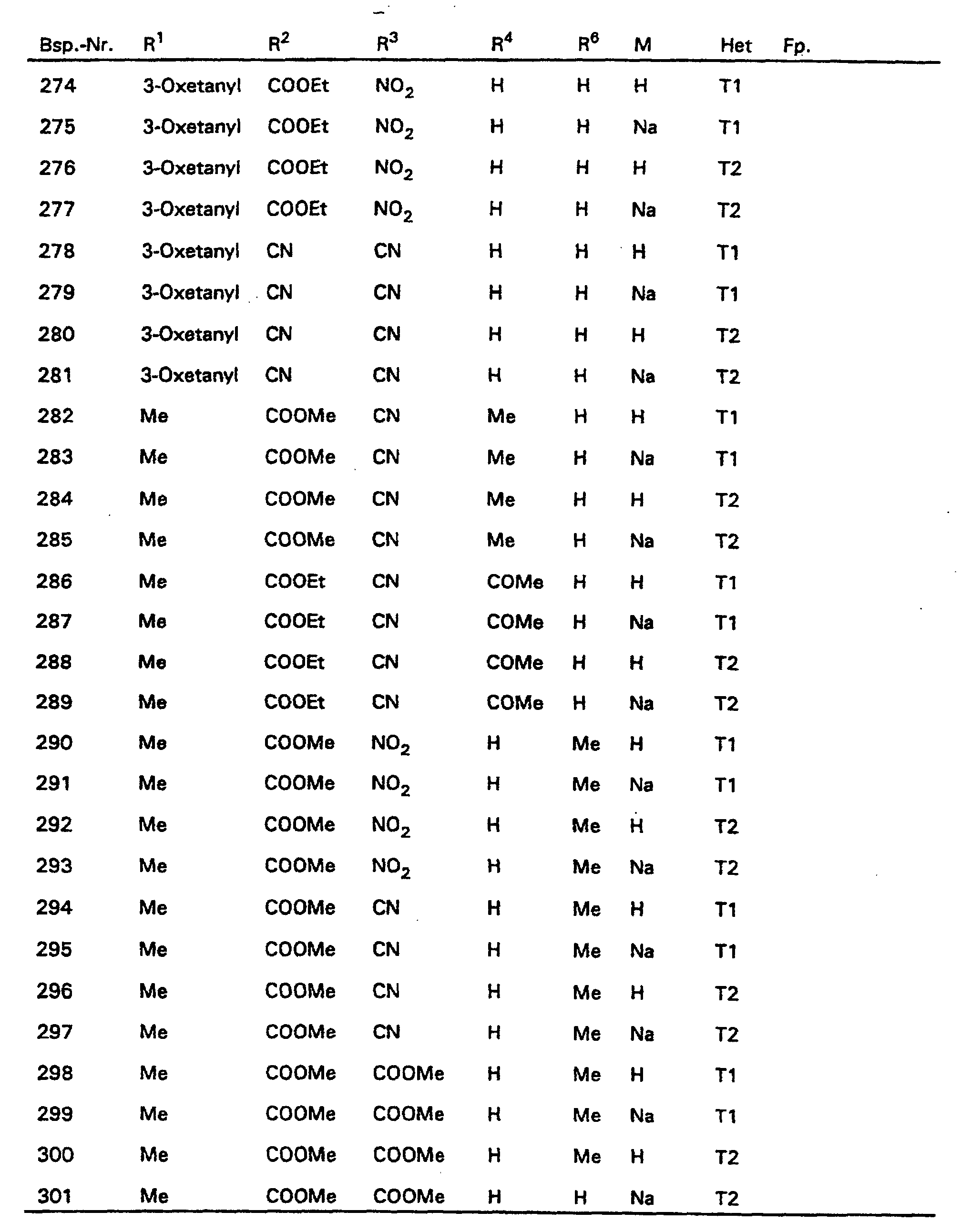

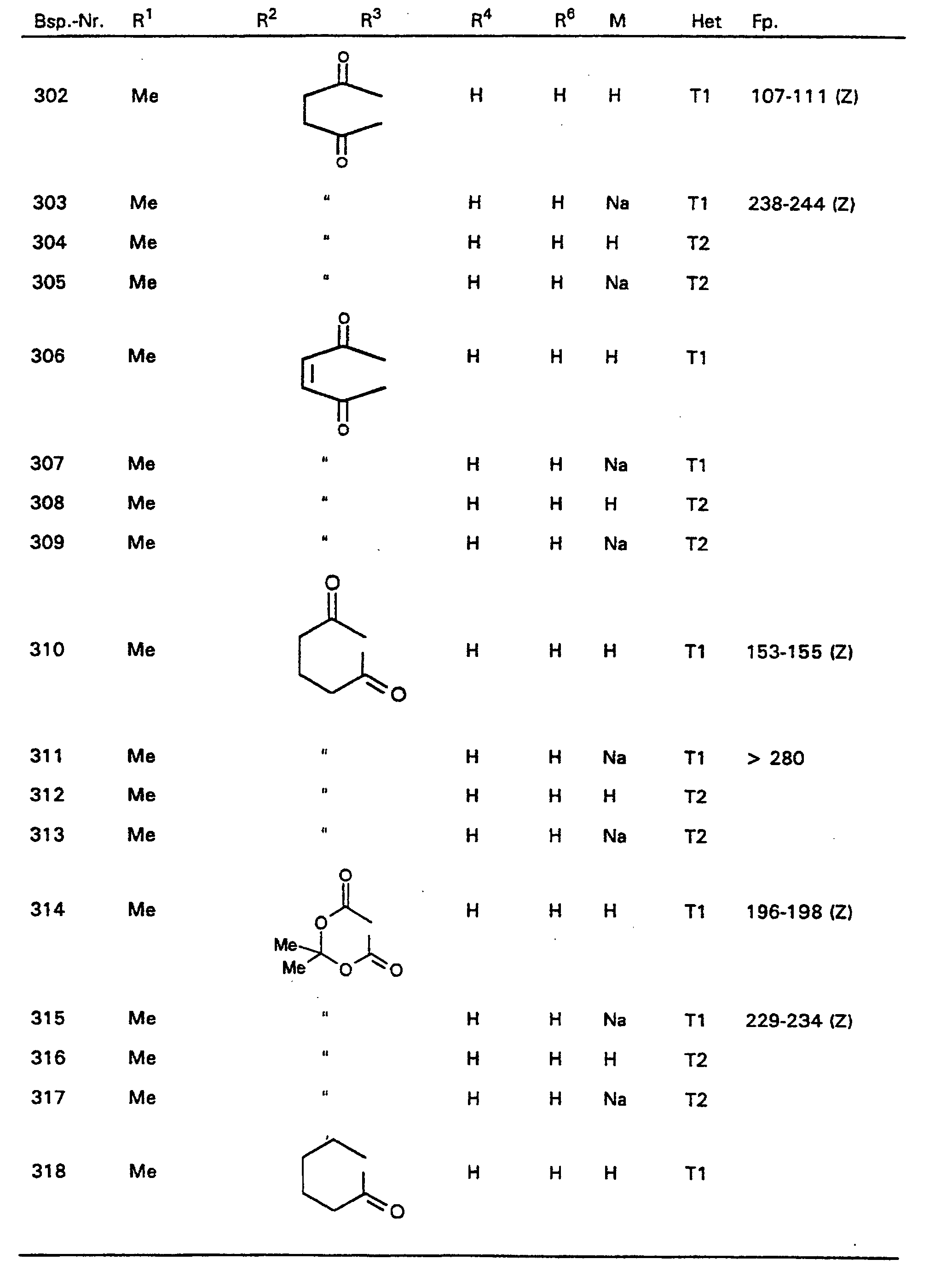

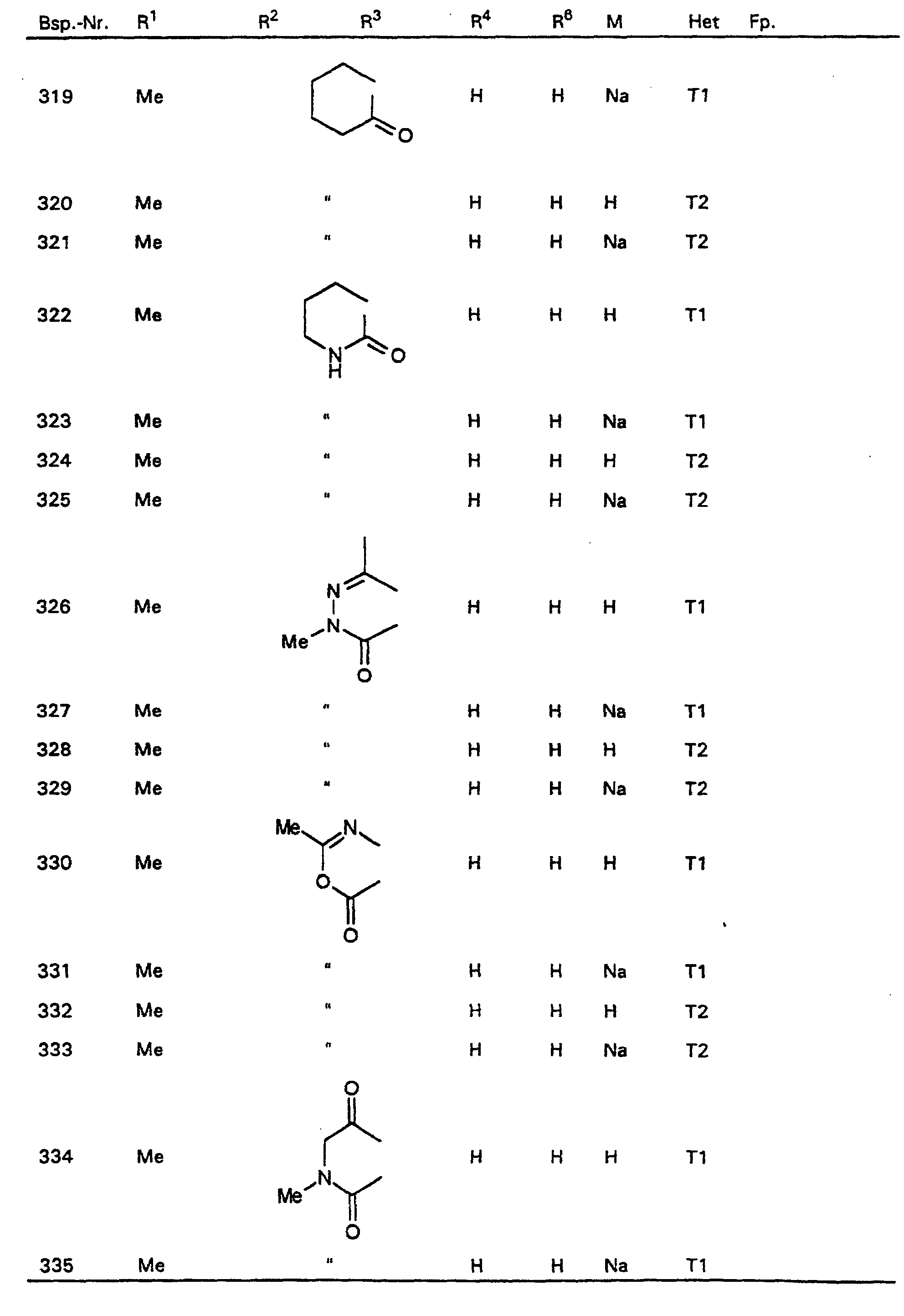

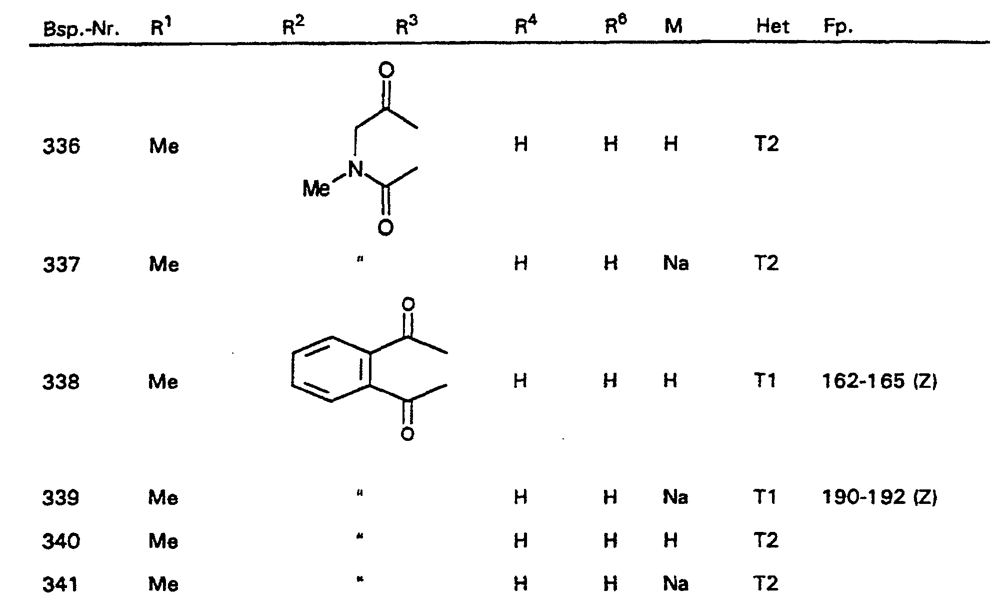

### B. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277°C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gewichtsteile einer Verbindung der Formel (I),
   10 Gewichtsteile ligninsulfonsaures Calcium,
   5 Gewichtsteile Natriumlaurylsulfat,
   3 Gewichtsteile Polyvinylalkohol und
   7 Gewichtsteile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gewichtsteile einer Verbindung der Formel (I),
   5 Gewichtsteile 2,2'-dinaphthytmethan-6,6'-disulfonsaures Natrium
   2 Gewichtsteile oleoylmethyltaurinsaures Natrium,
   1 Gewichtsteil Polyvinylalkohol,
   17 Gewichtsteile Calciumcarbonat und
   50 Gewichtsteile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Unkrautwirkung im Vorauflauf

Samen bzw. Rhizomstücke von mono- und dikotylen Unkrautpflanzen werden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen werden dann als wäßrige Suspension bzw. Emulsion mit einer Wasseraufwandmenge von umgerechnet 600 bis 800 l/ha in unterschiedlichen Dosierungen auf die Oberfläche der Abdeckerde appliziert.

Nach der Behandlung werden die Töpfe im Gewächshaus aufgestellt und unter guten Wachstumsbedingungen für die Unkräuter gehalten. Die optische Bonitur der Pflanzen- bzw. Auflaufschäden erfolgt nach dem Auflaufen der Versuchspflanzen nach einer Versuchstzeit von 3 bis 4 Wochen im Vergleich zu unbehandelten Kontrollen. Wie die Testergebnisse zeigen, weisen die erfindungsgemäßen Verbindungen eine gute herbizide Vorauflaufwirksamkeit gegen ein breites Spektrum von Ungräsern und Unkräutern auf. Beispielsweise haben die Beispiele: 5, 6, 17, 18, 42, 43, 46, 47, 58, 59, 67, 74, 75, 112, 113, 150, 151, 166, 186, 187, 198, 215, 302, 303, 310, 311, 314, 315, 338 und 339 (s. Tabelle 1) sehr gute herbizide Wirkung gegen Schadpflanzen wie Sinapis alba, Chrysanthemum segetum, Avena sativa, Stellaria media, Echinochloa crus-galli, Lolium multiflorum, Setaria spp., Abutilon theophrasti, Amaranthus retroflexus und Panicum miliaceum im Vorauflaufverfahren bei einer Aufwandmenge von 0,3 kg und weniger Aktivsubstanz pro Hektar.

### 2. Unkrautwirkung im Nachauflauf

Samen bzw. Rhizomstücke von mono- und dikotylen Unkräutern werden in Plastiktöpfen in sandigem Lehmboden ausgelegt, mit Erde abgedeckt und im Gewächshaus unter guten Wachstumsbedingungen angezogen. Drei Wochen nach der Aussaat werden die Versuchspflanzen im Dreiblattstadium behandelt. Die als Spritzpulver bzw. als Emulsionskonzentrate formulierten erfindungsgemäßen Verbindungen werden in verschiedenen Dosierungen mit einer Wasseraufwandmenge von umgerechnet 600 bis 800 l/ha auf die grünen Pflanzenteile gesprüht. Nach ca. 3 bis 4 Wochen Standzeit der Versuchspflanzen im Gewächshaus unter optimalen Wachstumsbedingungen wird die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise haben die Beispiele 5, 6, 17, 18, 42, 43, 46, 47, 58, 59, 67, 74, 75, 112, 113, 150, 151, 166, 186, 187, 198, 215, 302, 303, 310, 311, 314, 315, 338 und 339 (s. Tabelle 1) sehr gute herbizide Wirkung gegen Schadpflanzen wie Sinapis alba, Echinochloa crus-galli, Lolium multiflorum, Chrysanthemum segetum, Setaria spp., Abutilon theophrasti, Amaranthus retroflexus, Panicum miliaceum und Avena sativa im Nachauflaufverfahren bei einer Aufwandmenge von 0,3 kg und weniger Aktivsubstanz pro Hektar.

### 3. Kulturpflanzenverträglichkeit

In weiteren Versuchen im Gewächshaus werden Samen einer größeren Anzahl von Kulturpflanzen und Unkräutern in sandigem Lehmboden ausgelegt und mit Erde abgedeckt. Ein Teil der Töpfe wird sofort wie unter Abschnitt 1 beschrieben behandelt, die übrigen im Gewächshaus aufgestellt, bis die Pflanzen zwei bis drei echte Blätter entwickelt haben und dann wie unter Abschnitt 2 beschrieben mit den erfindungsgemäßen Substanzen der Formel (I) in unterschiedlichen Dosierungen besprüht. Vier bis fünf Wochen nach der Applikation und Standzeit im Gewächshaus wird mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vorund Nachauflaufverfahren selbst bei hohen Wirkstoffdosierungen ungeschädigt ließen. Einige Substanzen schonen darüber hinaus auch Gramineen-Kulturen wie z.B. Gerste, Weizen, Roggen, Hirsen, Mais oder Reis. Die Verbindungen der Formel (I) zeigen teilweise eine hohe Selektivität und eignen sich deshalb zur Bekämpfung von unerwünschten Pflanzenwuchs in landwirtschaftlichen Kulturen.

## Patentansprüche

1. Verbindungen der Formel (I) oder deren Salze, 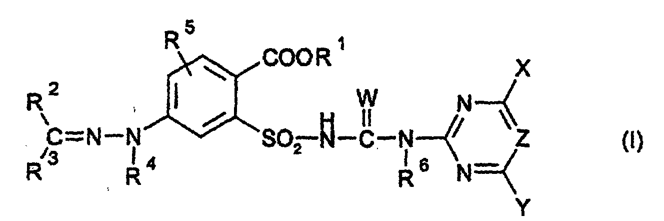 worin
R¹ H, (C₁-C₆)Alkyl, (C₃-C₆)Alkenyl, (C₃-C₆)Alkinyl, wobei jeder der drei letztgenannten Reste unsubstituiert oder durch einen oder mehrere Reste aus der Gruppe Halogen, Phenyl, (C₁-C₄)Alkoxy, (C₁-C₄)Alkylthio und [(C₁-C₄)Alkoxy]-carbonyl substituiert ist, oder (C₃-C₆)Cycloalkyl, (C₃-C₆)Cycloalkyl(C₁-C₃)alkyl, 3-Oxetanyl, wobei jeder der letztgenannten 3 Reste unsubstituiert oder durch einen oder mehrere Reste aus der Gruppe Halogen, (C₁-C₄)Alkyl und (C₁-C₄)Alkoxy substituiert ist,
a)
R² CN, NO₂ oder Acyl und
R³ CN, NO₂, Acyl, CF₃, oder Aryl, welches unsubstituiert oder substituiert ist durch einen oder mehrere Reste aus der Gruppe Oxo, Halogen, Alkoxy, Haloalkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, Mono- und Dialkylamino, Acylamino, Arylamino, N-Alkyl-N-Arylamino, Alkylsulfinyl, Haloalkylsulfinyl, Alkylsulfonyl, Haloalkylsulfonyl, Alkyl oder Haloalkyl sowie den genannten gesättigten kohlenwasserstoffhaltigen Resten entsprechende ungesättigte aliphatische Reste, oder
b)
R²R³C gemeinsam einen carbocyclischen oder heterocyclischen Ring, der in mindestens einer Nachbarstellung zu dem C-Atom in Position 1 der Gruppe CR²R³ ein C-Atom aufweist, das durch eine Oxogruppe substituiert ist, oder
c)
R² NO₂ oder [(C₁-C₄)Alkyl]-carbonyl und
R³ H oder (C₁-C₄)Alkyl,
R⁴ H, einen unsubstituierten oder substituierten, aliphatischen Kohlenwasserstoffrest mit 1 bis 6 C-Atomen im Kohlenwasserstoffteil und mit einem oder mehreren Substituenten, wobei die Substituenten aus der Gruppe Halogen, (C₁-C₄)Alkoxy, (C₁-C₄)Alkylthio, (C₁-C₄)Alkylsulfonyl, [(C₁-C₄)Alkyl]carbonyl, [(C₁-C₄)-Alkoxy]carbonyl, CN, Phenyl, das unsubstituiert oder ein- oder mehrfach substituiert ist durch gleiche oder verschiedene Reste aus der Gruppe Halogen, (C₁-C₄)Alkyl, (C₁-C₄)Alkoxy, (C₁-C₄)Halogenalkyl, (C₁-C₄)Halogenalkoxy und Nitro, und (C₃-C₆)Cycloalkyl ausgewählt sind, oder (C₃-C₆)Alkenyl oder (C₃-C₆)Alkinyl oder einen Acylrest,
R⁵ H, Halogen, NO₂, CN, (C₁-C₄)Alkyl, (C₁-C₄)Alkoxy, [(C₁-C₄)Alkyl]-carbonyl oder [(C₁-C₄)Alkoxy]carbonyl, wobei jeder der letztgenannten vier Reste unsubstituiert oder im Alkylteil durch ein oder mehrere Halogenatome substituiert ist,
R⁶ H oder (C₁-C₄)Alkyl,
W ein Sauerstoff- oder Schwefelatom,
X, Y unabhängig voneinander H, Halogen, (C₁-C₄)Alkyl, (C₁-C₄)Alkoxy, (C₁-C₄)Alkylthio, wobei jeder der letztgenannten 3 Reste unsubstituiert oder durch einen oder mehrere Reste aus der Gruppe Halogen, (C₁-C₄)Alkoxy und (C₁-C₄)Alkylthio substituiert ist, oder Mono- oder Di-[(C₁-C₄)alkyl]-amino, (C₃-C₆)Cycloalkyl, (C₂-C₅)Alkenyl, (C₃-C₅)Alkinyl, (C₃-C₅)-Alkenyloxy oder (C₂-C₅)Alkinyloxy und
Z CH oder N
bedeuten.

2. Verbindungen oder deren Salze nach Anspruch 1, **dadurch gekennzeichnet, daß**
R¹ H, (C₁-C₆)Alkyl, (C₃-C₆)Alkenyl, (C₃-C₆)Alkinyl, wobei jeder der drei letztgenannten Reste unsubstituiert oder durch einen oder mehrere Reste aus der Gruppe Halogen, Phenyl, (C₁-C₄)Alkoxy, (C₁-C₄)Alkylthio und [(C₁-C₄)Alkoxy]-carbonyl substituiert ist, oder (C₃-C₆)Cycloalkyl, (C₃-C₆)Cycloalkyl(C₁-C₃)alkyl, Heterocyclyl mit 3 bis 6 Ringatomen oder Heterocyclyl-(C₁-C₃)alkyl mit 3 bis 6 Ringatomen, wobei jeder der letztgenannten 4 Reste unsubstituiert oder durch einen oder mehrere Reste aus der Gruppe Halogen, (C₁-C₄)Alkyl und (C₁-C₄)Alkoxy substituiert ist,
a)
R² [(C₁-C₄)Alkyl]carbonyl, das unsubstituiert oder durch einen oder mehrere Reste aus der Gruppe Halogen, (C₁-C₄)Alkoxy, (C₁-C₄)Alkylthio und Phenyl substituiert ist, oder [(C₁-C₄)Alkoxy]carbonyl, das unsubstituiert oder durch einen oder mehrere Reste aus der Gruppe Halogen, (C₁-C₄)Alkoxy, Phenoxy und Phenyl substituiert ist, oder CONR⁷R⁸, CHO, CN, NO₂ oder Phenylcarbonyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Alkylthio, CN und NO₂ substituiert ist, oder (C₁-C₄)Alkylsulfonyl, (C₁-C₄)Haloalkylsulfonyl oder -P(O)[O(C₁-C₄)Alkyl]₂ und
R³ wie unter R² definiert oder CF₃ oder Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Alkylthio, (C₁-C₄)Alkylsulfonyl, [(C₁-C₄)Alkoxy]carbonyl, CN und NO₂ substituiert ist, oder
b)
R²R³C gemeinsam einen Ring mit 5 bis 8 Ringatomen, der carbocylisch oder heterocyclisch mit ein oder zwei Heteroatomen aus der Gruppe N, O und S ist und der durch eine oder zwei Oxogruppen jeweils in alpha-Position vom C-Atom in Position 1 der Gruppe CR²R³ substituiert und zusätzlich nicht substituiert oder zusätzlich durch einen oder mehrere Reste aus der Gruppe (C₁-C₄)Alkyl, (C₁-C₄)Haloalkyl, Halogen und Oxo substituiert ist, oder
c)
R² NO₂ oder [(C₁-C₄)Alkyl]carbonyl und
R³ H oder (C₁-C₄)Alkyl,
R⁴ H oder (C₁-C₆)Alkyl, das unsubstituiert oder durch einen oder mehrere Reste aus der Gruppe Halogen, (C₁-C₄)Alkoxy, (C₁-C₄)Alkylthio, (C₁-C₄)Alkylsulfonyl, [(C₁-C₄)Alkoxy]carbonyl, CN, Phenyl und (C₃-C₆)Cycloalkyl substituiert ist, oder (C₃-C₆)Alkenyl oder (C₃-C₆)Alkinyl, wobei jeder der zwei letztgenannten Reste unsubstituiert oder durch ein oder mehrere Halogenatome substituiert ist, oder eine Gruppe der Formel 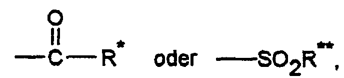 worin
R* H, (C₁-C₈)Alkyl, (C₂-C₆)Alkenyl, (C₂-C₆)Alkinyl, wobei jeder der drei letztgenannten Reste unsubstituiert oder durch einen oder mehrere Reste aus der Gruppe Halogen, (C₁-C₄)Alkoxy, (C₁-C₄)Alkylthio, Phenoxy, [(C₁-C₄)Alkoxy]carbonyl, unsubstituiertes und substituiertes Heterocyclyl und unsubstituiertes und substituiertes Phenyl substituiert ist, oder unsubstituiertes oder substituiertes (C₃-C₆)Cycloalkyl, unsubstituiertes oder substituiertes Phenyl, unsubstituiertes oder substituiertes Heterocyclyl oder [(C₁-C₄)Alkoxy]carbonyl,
R** (C₁-C₆)Alkyl, (C₃-C₆)Alkenyl, (C₃-C₆)Alkinyl, wobei jeder der drei letztgenannten Reste unsubstituiert oder durch einen oder mehrere Reste aus der Gruppe Halogen, (C₁-C₄)Alkoxy, (C₁-C₄)Alkylthio und Phenyl substituiert ist, oder Phenyl, das unsubstituiert oder durch einen oder mehrere Reste aus der Gruppe Halogen, CN, NO₂, (C₁-C₄)Alkyl, (C₁-C₄)Haloalkyl und (C₁-C₄)Alkoxy substituiert ist, bedeuten,
R⁷ und R⁸ unabhängig voneinander H, (C₁-C₄)Alkyl, (C₃-C₄)Alkenyl, (C₃-C₄)Alkinyl oder Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Alkylthio, (C₁-C₄)Alkylsulfonyl, [(C₁-C₄)Alkoxy]carbonyl, CN und NO₂ substituiert ist, oder
R⁷ und R⁸ gemeinsam mit dem N-Atom einen heterocyclischen Ring mit 5 oder 6 Ringgliedern, der gegebenenfalls weitere Heteroatome aus der Gruppe N, O oder S enthalten kann und unsubstituiert oder ein- oder mehrfach durch (C₁-C₄)Alkyl oder eine Oxogruppe substituiert ist,
W O oder S,
X und Y unabhängig voneinander H, Halogen, (C₁-C₄)Alkyl, (C₁-C₄)Alkoxy, (C₁-C₄)Alkylthio, wobei jeder der drei letztgenannten Reste unsubstituiert oder durch einen oder mehrere Reste aus der Gruppe Halogen, (C₁-C₃)Alkoxy und (C₁-C₄)Alkylthio substituiert ist, Mono- oder Di[(C₁-C₄)alkyl]amino, (C₃-C₆)-Cycloalkyl, (C₃-C₅)Alkenyl, (C₃-C₅)Alkenyloxy oder (C₃-C₅)Alkinyloxy und
Z CH oder N bedeuten.

3. Verbindungen der Formel (I) oder deren Salze, **dadurch gekennzeichnet, daß**
R¹ (C₁-C₆)Alkyl, das unsubstituiert oder durch einen oder mehrere Reste aus der Gruppe Halogen und (C₁-C₄)Alkoxy substituiert ist, oder 3-Oxetanyl, (C₃-C₄)Alkenyl oder (C₃-C₄)Alkinyl,
a)
R² und R³ unabhängig voneinander [(C₁-C₄)Alkyl]carbonyl, [(C₁-C₄)Alkoxy]carbonyl, CONR⁷R⁸, CHO, CN, NO₂, Benzoyl oder (C₁-C₄)Alkylsulfonyl oder
b)
CR²R³ gemeinsam einen Ring mit 5 bis 8 Ringatomen, der carbocyclisch oder heterocyclisch mit ein oder zwei Heteroatomen aus der Gruppe N, O und S ist und der durch eine oder zwei Oxogruppen jeweils in alpha-Position zum C-Atom in Position 1 der Gruppe CR²R³ substituiert und zusätzlich nicht substituiert oder zusätzlich durch einen oder mehrere Reste aus der Gruppe (C₁-C₄)Alkyl, (C₁-C₄)Haloalkyl und Halogen substituiert ist, oder
c)
R² NO₂ oder [(C₁-C₄)Alkyl]carbonyl und
R³ H oder (C₁-C₄)Alkyl,
R⁴ H, (C₁-C₄)Alkyl, das unsubstituiert oder durch einen oder mehrere Reste aus der Gruppe Halogen, (C₁-C₄)Alkoxy, (C₁-C₄)Alkylthio, [(C₁-C₄)Alkoxy]carbonyl und Phenyl substituiert ist oder (C₃-C₄)Alkenyl oder (C₃-C₄)Alkinyl,
R⁵ H, (C₁-C₄)Alkyl, (C₁-C₄)Haloalkyl, (C₁-C₄)Alkoxy oder Halogen,
X und Y unabhängig voneinander (C₁-C₄)Alkyl, (C₁-C₄)Alkoxy, wobei jeder der letztgenannten zwei Reste unsubstituiert oder durch einen oder mehrere Halogenatome substituiert ist, oder (C₁-C₄)Alkylthio, Halogen oder Mono- oder Di[(C₁-C₂)alkyl]amino und
W ein Sauerstoffatom
bedeuten.

4. Verbindungen oder deren Salze nach einem der Ansprüche 1 bis 3, **dadurch gekennzeichnet, daß**
R¹ Methyl oder Ethyl,
R² und R³ unabhängig voneinander [(C₁-C₂)Alkoxy]carbonyl, CN oder NO₂ oder
CR²R³ gemeinsam einen Ring mit 5 oder 6 Ringatomen, der carbocyclisch oder heterocyclisch mit ein oder zwei Heteroatomen aus der Gruppe N, O und S ist und der durch eine oder zwei Oxogruppen jeweils in alpha-Position zum C-Atom in Position 1 der Gruppe CR²R³ substituiert und zusätzlich nicht substituiert oder zusätzlich durch einen oder mehrere (C₁-C₂)Alkylreste substituiert ist, und
R⁴ H,
R⁵ H, (C₁-C₄)Alkyl oder Halogen,
X (C₁-C₂)Alkyl, (C₁-C₂)Alkoxy, (C₁-C₂)Alkylthio, (C₁-C₂)Haloalkyl oder (C₁-C₂)Haloalkoxy und
Y (C₁-C₂)Alkyl, (C₁-C₂)Alkoxy, Halogen, NHCH₃ oder N(CH₃)₂
bedeuten.

5. Verfahren zur Herstellung von Verbindungen der Formel (I) oder deren Salze, wie sie nach einem der Ansprüche 1 bis 4 definiert sind, **dadurch gekennzeichnet, daß**
a) eine Verbindung der Formel (II) 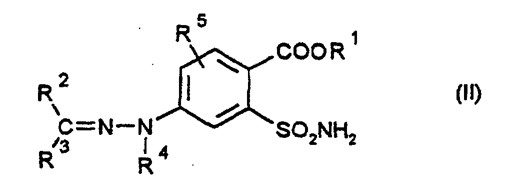 mit einem heterocyclischen Carbamat der Formel (III), 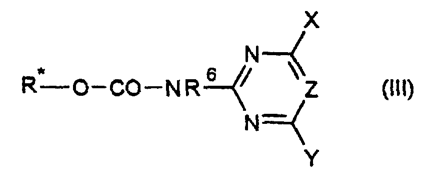 worin R* gegebenenfalls substituiertes Phenyl oder (C₁-C₄)Alkyl bedeutet, umsetzt oder
b) ein Sulfonylcarbamat der Formel (IV), 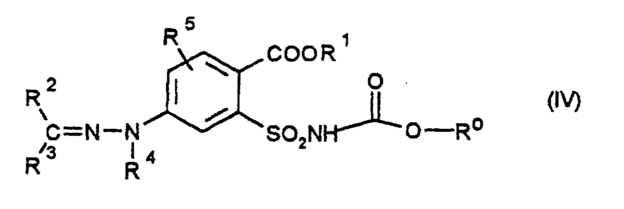 worin R^{o} gebenenfalls substituiertes Phenyl oder (C₁-C₄)Alkyl bedeutet, mit einem Aminoheterocyclus der Formel (V) 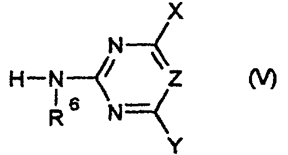 umsetzt oder
c) ein Sulfonylisocyanat der Formel (VI) 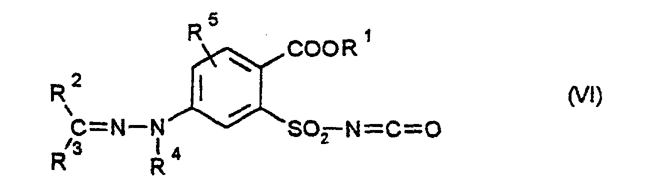 mit einem Aminoheterocyclus der Formel (V) umsetzt oder
d) ein Sulfonamid der Formel (II) mit einem (Thio)-Isocyanat der Formel (VII) 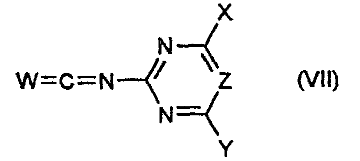 in Gegenwart einer Base umsetzt oder
e) einen Aminoheterocyclus der Formel (V) zunächst basenkatalysiert mit einem Kohlensäureester umsetzt und das gebildete Intermediat in einer Eintopfreaktion mit einem Sulfonamid der Formel (II) (siehe Variante a) umsetzt,
wobei in den Formeln (II)-(VII) die Reste bzw. Gruppen R¹-R⁶, W, X, Y und Z wie in Formel (I) definiert sind und in Verfahrensvarianten a) bis c) und e) zunächst Verbindungen (I) mit W = O erhalten werden.

6. Herbizides oder pflanzenwachstumsregulierende Mittel, **dadurch gekennzeichnet, daß** es mindestens eine Verbindung der Formel (I) oder deren Salz nach einem der Ansprüche 1 bis 4 und im Pflanzenschutz übliche Formulierungshilfsmittel enthält.

7. Verfahren zur Bekämpfung von Schadpflanzen oder zur Wachstumsregulierung von Pflanzen, **dadurch gekennzeichnet, daß** man eine wirksame Menge von mindestens einer Verbindung der Formel (I) oder deren Salz nach einem der Ansprüche 1 bis 4 auf die Schadpflanzen bzw. Pflanzen, deren Pflanzensamen oder die Fläche, auf der sie wachsen, appliziert.

8. Verwendung der Verbindungen der Formel (I) oder deren Salze nach einem der Ansprüche 1 bis 4 als Herbizide oder Pflanzenwachstumsregulatoren.

9. Verbindungen der Formel (II), (IV) und (VI) wie sie in Anspruch 5 definiert sind.

## Claims

1. A compound of the formula (I) or a salt thereof 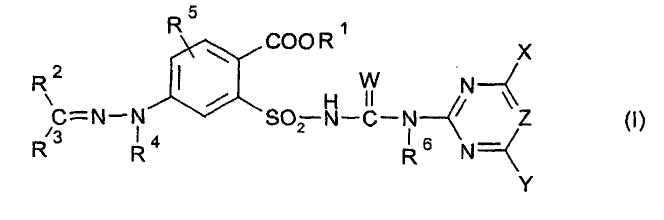 in which
R¹ is H, (C₁-C₆)alkyl, (C₃-C₆)alkenyl, (C₃-C₆)alkynyl, where each of the three last-mentioned radicals is unsubstituted or substituted by one or more radicals selected from the group consisting of halogen, phenyl (C₁-C₄)alkoxy, (C₁-C₄)alkylthio and [(C₁-C₄)alkoxy]carbonyl, or is (C₃-C₆)cycloalkyl, (C₃-C₆)cycloalkyl (C₁-C₃)alkyl, 3-oxetanyl, where each of the three last-mentioned radicals is unsubstituted or substituted by one or more radicals selected from the group consisting of halogen, (C₁-C₄)alkyl and (C₁-C₄)alkoxy,
a)
R² is CN, NO₂ or acyl and
R³ is CN, NO₂, acyl, CF₃, or aryl which is unsubstituted or substituted by one or more radicals selected from the group consisting of oxo, halogen, alkoxy, haloalkoxy, alkylthio, hydroxyl, amino, nitro, carboxyl, cyano, azido, alkoxycarbonyl, alkylcarbonyl, formyl, carbamoyl, mono- and dialkylaminocarbonyl, mono- and dialkylamino, acylamino, arylamino, N-alkyl-N-arylamino, alkylsulfinyl, haloalkylsulfinyl, alkylsulfonyl, haloalkylsulfonyl, alkyl or haloalkyl and unsaturated aliphatic radicals which correspond to the saturated hydrocarbon-containing radicals mentioned, or
b)
R²R³C together form a carbocyclic or heterocyclic ring which has a carbon atom substituted by an oxo group in at least one position adjacent to the carbon atom in position 1 of the group CR²R³, or
c)
R² is NO₂ or [(C₁-C₄)alkyl]carbonyl and
R³ is H or (C₁-C₄)alkyl,
R⁴ is H, an unsubstituted or substituted aliphatic hydrocarbon radical having 1 to 6 carbon atoms in the hydrocarbon moiety and one or more substituents, where the substituents are selected from the group consisting of halogen, (C₁-C₄)alkoxy, (C₁-C₄)alkylthio, (C₁-C₄)alkylsulfonyl, [(C₁-C₄)alkyl]carbonyl, [(C₁-C₄)alkoxy]-carbonyl, CN, phenyl, which is unsubstituted or mono- or polysubstituted by identical or different radicals selected from the group consisting of halogen, (C₁-C₄)alkyl, (C₁-C₄)alkoxy, (C₁-C₄)haloalkyl, (C₁-C₄)haloalkoxy and nitro, and (C₃-C₆)cycloalkyl, or is (C₃-C₆)alkenyl or (C₃-C₆)alkynyl or an acyl radical,
R⁵ is H, halogen, NO₂, CN, (C₁-C₄)alkyl, (C₁-C₄)alkoxy, [(C₁-C₄)alkyl]carbonyl or [(C₁-C₄)alkoxy]carbonyl, where each of the four last-mentioned radicals is unsubstituted or substituted in the alkyl moiety by one or more halogen atoms,
R⁶ is H or (C₁-C₄)alkyl,
W is an oxygen or sulfur atom,
X, Y independently of one another are H, halogen, (C₁-C₄)alkyl, (C₁-C₄)alkoxy, (C₁-C₄)alkylthio, where each of the 3 last-mentioned radicals is unsubstituted or substituted by one or more radicals selected from the group consisting of halogen, (C₁-C₄)alkoxy and (C₁-C₄)alkylthio, or is mono- or di[(C₁-C₄)alkyl]amino, (C₃-C₆)cycloalkyl, (C₂-C₅)alkenyl, (C₃-C₅)alkynyl, (C₃-C₅)alkenyloxy or (C₂-C₅)alkynyloxy and
Z is CH or N.

2. The compound or a salt thereof as claimed in claim 1, wherein
R¹ is H, (C₁-C₆)alkyl, (C₃-C₆)alkenyl, (C₃-C₆)alkynyl, where each of the three last-mentioned radicals is unsubstituted or substituted by one or more radicals selected from the group consisting of halogen, phenyl, (C₁-C₄)alkoxy, (C₁-C₄)alkylthio and [(C₁-C₄)alkoxy]carbonyl, or is (C₃-C₆)cycloalkyl, (C₃-C₆)cycloalkyl(C₁-C₃)alkyl, heterocyclyl having 3 to 6 ring atoms or heterocyclyl(C₁-C₃)alkyl having 3 to 6 ring atoms, where each of the 4 last-mentioned radicals is unsubstituted or substituted by one or more radicals selected from the group consisting of halogen, (C₁-C₄)alkyl and (C₁-C₄)alkoxy,
a)
R² is [(C₁-C₄)alkyl]carbonyl which is unsubstituted or substituted by one or more radicals selected from the group consisting of halogen, (C₁-C₄)alkoxy, (C₁-C₄)alkylthio and phenyl, or is [(C₁-C₄)alkoxy]carbonyl which is unsubstituted or substituted by one or more radicals selected from the group consisting of halogen, (C₁-C₄)alkoxy, phenoxy and phenyl, or is CONR⁷R⁸, CHO, CN, NO₂ or phenylcarbonyl which is unsubstituted or substituted by one or more radicals selected from the group consisting of halogen, (C₁-C₄)alkyl, (C₁-C₄)haloalkyl, (C₁-C₄)alkoxy, (C₁-C₄)alkylthio, CN and NO₂, or is (C₁-C₄)alkylsulfonyl, (C₁-C₄)haloalkylsulfonyl or -P(O)[O(C₁-C₄)alkyl]₂ and
R³ is as defined under R² or is CF₃ or phenyl which is unsubstituted or substituted by one or more radicals selected from the group consisting of halogen, (C₁-C₄)alkyl, (C₁-C₄)haloalkyl, (C₁-C₄)alkoxy, (C₁-C₄)alkylthio, (C₁-C₄)alkylsulfonyl, [(C₁-C₄)alkoxy]carbonyl, CN and NO₂, or
b)
R²R³C together form a ring having 5 to 8 ring atoms, which is carbocyclic or heterocyclic and has one or two heteroatoms selected from the group consisting of N, O and S and which is substituted by one or two oxo groups, in each case in the position alpha to the carbon atom in position 1 of the group CR²R³ and additionally unsubstituted or additionally substituted by one or more radicals selected from the group consisting of (C₁-C₄)alkyl, (C₁-C₄)haloalkyl, halogen and oxo, or
c)
R² is NO₂ or [(C₁-C₄)alkyl]carbonyl and
R³ is H or (C₁-C₄)alkyl,
R⁴ is H or (C₁-C₆)alkyl which is unsubstituted or substituted by one or more radicals selected from the group consisting of halogen, (C₁-C₄)alkoxy, (C₁-C₄)alkylthio, (C₁-C₄)alkylsulfonyl, [(C₁-C₄)alkoxy]carbonyl, CN, phenyl and (C₃-C₆)cycloalkyl, or is (C₃-C₆)alkenyl or (C₃-C₆)alkynyl, where each of the two last-mentioned radicals is unsubstituted or substituted by one or more halogen atoms, or is a group of the formula 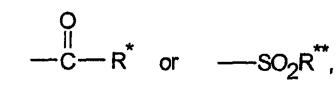 in which
R* is H, (C₁-C₈)alkyl, (C₂-C₆)alkenyl, (C₂-C₆)alkynyl, where each of the three last-mentioned radicals is unsubstituted or substituted by one or more radicals selected from the group consisting of halogen, (C₁-C₄)alkoxy, (C₁-C₄)alkylthio, phenoxy, [(C₁-C₄)alkoxy]carbonyl, unsubstituted and substituted heterocyclyl and unsubstituted and substituted phenyl, or is unsubstituted or substituted (C₃-C₆)cycloalkyl, unsubstituted or substituted phenyl, unsubstituted or substituted heterocyclyl or [(C₁-C₄)alkoxy]carbonyl,
R** is (C₁-C₆)alkyl, (C₃-C₆)alkenyl, (C₃-C₆)alkynyl, where each of the three last-mentioned radicals is unsubstituted or substituted by one or more radicals selected from the group consisting of halogen, (C₁-C₄)alkoxy, (C₁-C₄)alkylthio and phenyl, or is phenyl which is unsubstituted or substituted by one or more radicals selected from the group consisting of halogen, CN, NO₂, (C₁-C₄)alkyl, (C₁-C₄)haloalkyl and (C₁-C₄)alkoxy,
R⁷ and R⁸ independently of one another are H, (C₁-C₄)alkyl, (C₃-C₄)alkenyl, (C₃-C₄)alkynyl or phenyl which is unsubstituted or substituted by one or more radicals selected from the group consisting of halogen, (C₁-C₄)alkyl, (C₁-C₄)haloalkyl, (C₁-C₄)alkoxy, (C₁-C₄)alkylthio, (C₁-C₄)alkylsulfonyl, [(C₁-C₄)alkoxy]carbonyl, CN and NO₂, or
R⁷ and R⁸ together with the nitrogen atom form a heterocyclic ring having 5 or 6 ring members, which may, if appropriate, contain further heteroatoms selected from the group consisting of N, O and S and which is unsubstituted or mono- or polysubstituted by (C₁-C₄)alkyl or an oxo group,
W is O or S,
X and Y independently of one another are H, halogen, (C₁-C₄)alkyl, (C₁-C₄)alkoxy, (C₁-C₄)alkylthio, where each of the three last-mentioned radicals is unsubstituted or substituted by one or more radicals selected from the group consisting of halogen, (C₁-C₃)alkoxy and (C₁-C₄)alkylthio, mono- or di[(C₁-C₄)alkyl]amino, (C₃-C₆)cycloalkyl, (C₃-C₅)alkenyl, (C₃-C₅)alkenyloxy or (C₃-C₅)alkynyloxy and
Z is CH or N.

3. A compound or a salt thereof as claimed in claim 1 or 2, wherein
R¹ is (C₁-C₆)alkyl which is unsubstituted or substituted by one or more radicals selected from the group consisting of halogen and (C₁-C₄)alkoxy, or is 3-oxetanyl, (C₃-C₄)alkenyl or (C₃-C₄)alkynyl,
a)
R² and R³ independently of one another are [(C₁-C₄)alkyl]carbonyl, [(C₁-C₄)alkoxy]carbonyl, CONR⁷R⁸, CHO, CN, NO₂, benzoyl or (C₁-C₄)alkylsulfonyl or
b)
CR²R³ together form a ring having 5 to 8 ring atoms, which is carbocyclic or heterocyclic and has one or two heteroatoms selected from the group consisting of N, O and S and which is substituted by one or two oxo groups, in each case in the position alpha to the carbon atom in position 1 of the group CR²R³ and additionally unsubstituted or additionally substituted by one or more radicals selected from the group consisting of (C₁-C₄)alkyl, (C₁-C₄)haloalkyl and halogen, or
c)
R² is NO₂ or [(C₁-C₄)alkyl]carbonyl and
R³ is H or (C₁-C₄)alkyl,
R⁴ is H, (C₁-C₄)alkyl which is unsubstituted or substituted by one or more radicals selected from the group consisting of halogen, (C₁-C₄)alkoxy, (C₁-C₄)alkylthio, [(C₁-C₄)alkoxy]carbonyl and phenyl or is (C₃-C₄)alkenyl or (C₃-C₄)alkynyl,
R⁵ is H, (C₁-C₄)alkyl, (C₁-C₄)haloalkyl, (C₁-C₄)alkoxy or halogen,
X and Y independently of one another are (C₁-C₄)alkyl, (C₁-C₄)alkoxy, where each of the two last-mentioned radicals is unsubstituted or substituted by one or more halogen atoms, or are (C₁-C₄)alkylthio, halogen or mono- or di[(C₁-C₂)alkyl]amino and
W is an oxygen atom.

4. The compound or a salt thereof as claimed in any of claims 1 to 3, wherein
R¹ is methyl or ethyl,
R² and R³ independently of one another are [(C₁-C₂)alkoxy]carbonyl, CN or NO₂ or
CR²R³ together form a ring having 5 or 6 ring atoms, which is carbocyclic or heterocyclic and has one or two heteroatoms selected from the group consisting of N, O and S and which is substituted by one or two oxo groups, in each case in the position alpha to the carbon atom in position 1 of the group CR²R³, and which is additionally unsubstituted or additionally substituted by one or more (C₁-C₂)alkyl radicals, and
R⁴ is H,
R⁵ is H, (C₁-C₄)alkyl or halogen,
X is (C₁-C₂)alkyl, (C₁-C₂)alkoxy, (C₁-C₂)alkylthio, (C₁-C₂)haloalkyl or (C₁-C₂)haloalkoxy and
Y is (C₁-C₂)alkyl, (C₁-C₂)alkoxy, halogen, NHCH₃ or N(CH₃)₂.

5. A process for preparing compounds of the formula (I) or salts thereof as defined in any of claims 1 to 4, which comprises
a) reacting a compound of the formula (II) 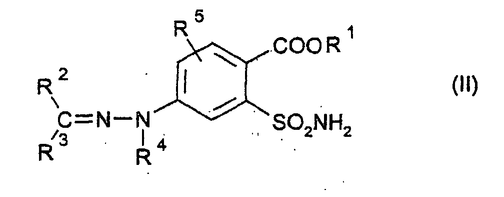 with a heterocyclic carbamate of the formula (III), 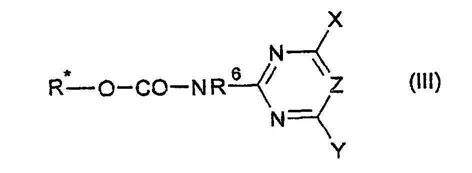 in which R* is phenyl with or without substitution or (C₁-C₄)alkyl, or
b) reacting a sulfonylcarbamate of the formula (IV), 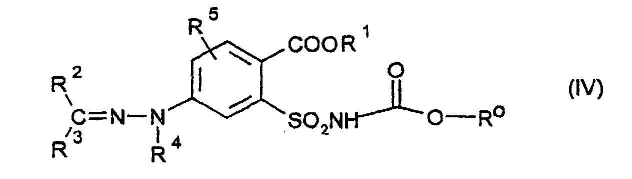 in which R^{o} is phenyl with or without substitution or (C₁-C₄)alkyl, with an aminoheterocycle of the formula (V) 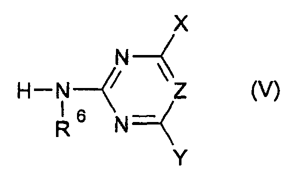 or
c) reacting a sulfonyl isocyanate of the formula (VI) 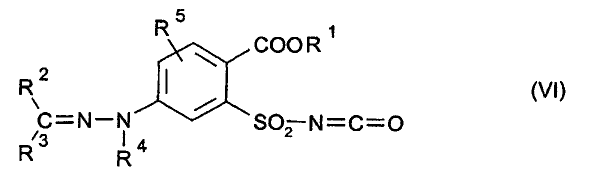 with an aminoheterocycle of the formula (V) or
d) reacting a sulfonamide of the formula (II) with a (thio)isocyanate of the formula (VII) 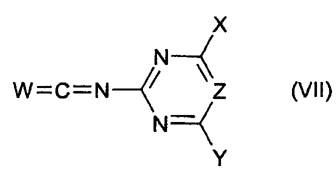 in the presence of a base or
e) reacting an aminoheterocycle of the formula (V) initially with a carbonic ester under base catalysis and reacting the formed intermediate in a one-pot reaction with a sulfonamide of the formula (II) (see variant a), where in the formulae (II)-(VII), the radicals or groups R¹-R⁶, W, X, Y and Z are as defined in formula (I) and in process variants a) to c) and e) compounds (I) where W = O are initially obtained.

6. A herbicidal or plant-growth-regulating composition, which comprises at least one compound of the formula (I) or a salt thereof as claimed in any of claims 1 to 4 and formulation auxiliaries customary in crop protection.

7. A method for controlling harmful plants or for regulating the growth of plants, which comprises applying an effective amount of at least one compound of the formula (I) or a salt thereof as claimed in any of claims 1 to 4 to the harmful plants or plants, to seeds thereof or to the area where they grow.

8. The use of the compounds of the formula (I) or salts thereof as claimed in any of claims 1 to 4 as herbicides or plant growth regulators.

9. A compound of the formula (II), (IV) and (VI) as defined in claim 5.

## Revendications

1. Composés de formule (I) ou leurs sels 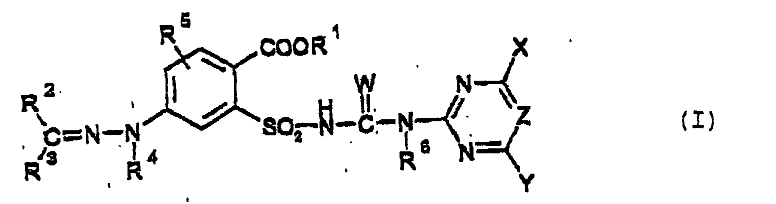 où
R¹ représente un atome d'hydrogène, des groupes alkyle en C₁-C₆, alcényle en C₃-C₆, alcynyle en C₃-C₆, chacun des trois derniers restes cités est non substitué ou substitué par un ou plusieurs restes pris dans le groupe comprenant des restes halogène, phényle, alkoxy en C₁-C₄, (alkyl en C₁-C₄)-thio et (alkoxy en C₁-C₄)carbonyle, ou cycloalkyle en C₃-C₆, (cycloalkyle en C₃-C₆)(alkyle en C₁-C₃), 3-oxétanyle, les trois derniers restes cités étant non substitués ou substitués par un ou plusieurs restes pris dans le groupe comprenant des restes halogène, alkyle en C₁-C₄ et alkoxy en C₁-C₄,
a)
R² représente des groupes CN, NO₂ ou acyle et
R³ représente des groupes CN, NO₂, acyle, CF₃ ou aryle, qui est non substitué ou substitué par un ou plusieurs restes pris dans le groupe comprenant oxo, halogène, alkoxy, haloalkoxy, alkylthio, hydroxy, amino, nitro, carboxy, cyano, azido, alkoxycarbonyle, alkylcarbonyle, formyle, carbamoyle, mono- et dialkylaminocarbonyle, mono- et dialkylamino, acylamino, arylamino, N-alkyl-N-arylamino, alkylsulfinyle, haloalkylsulfinyle, alkylsulfonyle, haloalkylsulfonyle, alkyle ou haloalkyle, ainsi que les restes aliphatiques insaturés correspondant aux restes hydrocarbonés saturés, ou
b)
R²R³C conjointement forment un cycle carbocylique ou hétérocyclique, qui présente, dans au moins une position adjacente à l'atome de carbone en position 1 du groupe CR²R³, un atome de carbone, qui est substitué par un groupe oxo, ou
c)
R² représente des groupes NO₂ ou (alkyl en C₁-C₄)-carbonyle et
R³ représente un atome d'hydrogène ou un groupe alkyl en C₁-C₄,
R⁴ représente un atome d'hydrogène, un reste hydrocarboné aliphatique, non substitué ou substitué, ayant 1 à 6 atomes de carbone dans le fragment hydrocarboné et présente un ou plusieurs substituants, les substituants étant pris dans le groupe comprenant halogènes, alkoxy en C₁-C₄, (alkyl en C₁-C₄)thio, (alkyl en C₁-C₄)sulfonyle, (alkyl en C₁-C₄)carbonyle, (alkoxy en C₁-C₄)carbonyle, CN, phényle, qui est non substitué ou substitué une ou plusieurs fois par des restes identiques ou différents pris dans le groupe comprenant des substituants halogène, alkyle en C₁-C₄, alkoxy en C₁-C₄, haloalkyle en C₁-C₄, haloalkoxy, en C₁-C₄, et nitro, et cycloalkyle en C₃-C₆, ou alcényle en C₃-C₆ ou alcynyle en C₃-C₆ ou un reste acyle,
R⁵ représente un atome d'hydrogène, un atome d'halogène, des groupes NO₂, CN, alkyle en C₁-C₄, alkoxy en C₁-C₄, (alkyl en C₁-C₄)carbonyle ou (alkoxy en C₁-C₄)carbonyle, chacun des quatre derniers restes cités pouvant être non substitué ou substitué dans le fragment alkyle par un ou plusieurs atomes d'halogènes,
R⁶ représente un atome d'hydrogène ou un groupe alkyle en C₁-C₄,
W représente un atome d'oxygène ou un atome de soufre,
X, Y représentent, indépendamment l'un de l'autre, des atomes d'hydrogène, des atomes d'halogènes, des groupes alkyle en C₁-C₄, alkoxy en C₁-C₄, (alkyl en C₁-C₄)thio, chacun des trois derniers restes cités étant non substitué ou substitué par un ou plusieurs restes pris dans le groupe comprenant des atomes d'halogènes, des groupes alkoxy en C₁-C₄ et (alkyl en C₁-C₄)thio, ou des groupes mono- ou di-(alkyl en C₁-C₄)amino, cycloalkyle en C₃-C₃, alcényle en C₂-C₅, alcynyle en C₃-C₅, alcényloxy en C₃-C₅ ou alcynyloxy en C₂-C₅ et
Z représente des groupes CH ou N.

2. Composés ou leurs sels selon la revendication 1, **caractérisés en ce que**
R¹ représente un atome d'hydrogène, des groupes alkyle en C₁-C₆, alcényle en C₃-C₆, alcynyle en C₃-C₆, chacun des trois derniers restes cités étant non substitué ou substitué par un ou plusieurs restes pris dans le groupe comprenant des restes halogéne, phényle, alkoxy en C₁-C₄, (alkyl en C₁-C₄)thio et (alkoxy en C₁-C₄)carbonyle, ou représente cycloalkyle en C₃-C₆, (cycloalkyle en C₃-C₆)-(alkyle en C₁-C₃), hétérocyclyle présentant 3 à 6 atomes cycliques ou hétérocyclyl-(alkyle en C₁-C₃) présentant 3 à 6 atomes cycliques, chacun des quatre derniers restes cités étant non substitués ou substitués par un ou plusieurs restes pris dans le groupe comprenant des restes halogène, alkyle en C₁-C₄ et alkoxy en C₁-C₄,
a)
R² représente un groupe (alkyl en C₁-C₄)carbonyle qui est non substitué ou substitué par un ou plusieurs restes pris dans le groupe comprenant des atomes d'halogènes, des groupes alkoxy en C₁-C₄, (alkyl en C₁-C₄)thio et phényle, ou un groupe (alkoxy en C₁-C₄)carbonyle qui est non substitué ou substitué par un ou plusieurs restes pris dans le groupe comprenant des atomes d'halogènes, des groupes alkoxy en C₁-C₄, phénoxy et phényle, ou un groupe CONR⁷R⁸, CHO, CN, NO₂ ou phénylcarbonyle, qui est non substitué ou substitué par un ou plusieurs restes pris dans le groupe comprenant des atomes d'halogènes, des groupes alkyle en C₁-C₄, haloalkyle en C₁-C₄, alkoxy en C₁-C₄, (alkyl en C₁-C₄)thio, CN et NO₂, ou (alkyl en C₁-C₄)sulfonyle ou halo(alkyl en C₁-C₄)sulfonyle ou -P(O)[O(alkyl en C₁-C₄]₂ et
R³ est défini comme à R² ou représente des groupes CF₃ ou phényle, qui est non substitué ou substitué par un ou plusieurs restes pris dans le groupe comprenant des atomes d'halogènes, des groupes alkyle en C₁-C₄, haloalkyle en C₁-C₄, alkoxy en C₁-C₄, (alkyl en C₁-C₄)thio, (alkyl en C₁-C₄)sulfonyle, (alkoxy en C₁-C₄)carbonyle, CN et NO₂
b)
R²R³C représentent ensemble un cycle de 5 à 8 atomes cycliques, qui est carbocylique ou hétérocyclique, substitué par un ou deux hétéroatomes pris dans le groupes des atomes de N, O et S et par un ou deux groupes oxo chaque fois en position alpha par rapport à l'atome de carbone en position 1 du groupe CR²R³ et n'est pas substitué en plus ou est substitué en plus par un ou plusieurs restes pris dans le groupe comprenant alkyle en C₁-C₄, haloalkyle en C₁-C₄, halogène et oxo, ou
c)
R² représente des groupes NO₂ ou (alkyl en C₁-C₄)carbonyle et
R³ représente un atome d'hydrogène ou un groupe alkyle en C₁-C₄,
R⁴ représente un atome d'hydrogène ou un groupe alkyle en C₁-C₆, qui est non substitué ou substitué par un ou plusieurs restes pris dans le groupe comprenant des atomes d'halogènes, des groupes alkoxy en C₁-C₄, (alkyl en C₁-C₄)thio, (alkyl en C₁-C₄) sulfonyle, (alkoxy en C₁-C₄)carbonyle, CN, phényle et cycloalkyle en C₃-C₆, ou alcényle en C₃-C₆ ou alcynyle en C₃-C₆, chacun des deux derniers restes cités étant non substitué ou substitué par un ou plusieurs atomes d'halogènes, ou un groupe de formule 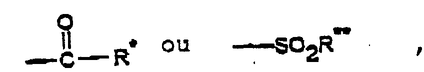 où
R* représente un atome d'hydrogène, des groupes alkyle en C₁-C₈, alcényle en C₂-C₆, alcynyle en C₂-C₆, chacun des trois derniers restes cités étant non substitué ou substitué par un ou plusieurs restes pris dans le groupe comprenant des atomes d'halogènes, des groupes alkoxy en C₁-C₄, (alkyl en C₁-C₄)thio, phénoxy, (alkoxy en C₁-C₄)carbonyle, hétérocyclyle non substitué et substitué et phényle non substitué ou substitué, ou représente cycloalkyle en C₃-C₆ non substitué ou substitué, phényle non substitué ou substitué, hétérocyclyle non substitué ou substitué ou (alkoxy en C₁-C₄)carbonyle,
R** représente des groupes alkyle en C₁-C₆, alcényle en C₃-C₆, alcynyle en C₃-C₆, chacun des trois derniers restes cités étant non substitué ou substitué par un ou plusieurs restes pris dans le groupe comprenant des atomes d'halogènes, des groupes alkoxy en C₁-C₄, (alkyl en C₁-C₄)thio et phényle, ou phényle, qui est non substitué ou substitué par un ou plusieurs restes pris dans le groupe comprenant des atomes d'halogènes, des groupes CN, NO₂, alkyle en C₁-C₄, haloalkyle en C₁-C₄ et alkoxy en C₁-C₄,
R⁷ et R⁸ représentent, indépendamment l'un de l'autre, des atomes d'hydrogène, des groupes alkyle en C₁-C₄, alcényle en C₃-C₄, alcynyle en C₃-C₄ ou phényle, qui est non substitué ou substitué par un ou plusieurs restes pris dans le groupe comprenant des atomes d'halogènes, des groupes alkyle en C₁-C₄, haloalkyle en C₁-C₄, alkoxy en C₁-C₄, (alkyl en C₁-C₄)thio, (alkyl en C₁-C₄)sulfonyle, (alkoxy en C₁-C₄)carbonyle, CN et NO₂, ou
R⁷ et R⁸ représentent ensemble avec l'atome de N un cycle hétérocyclique de 5 ou 6 chaînons, qui peut éventuellement contenir d'autres hétéroatomes pris dans le groupe des atomes de N, O out S, et qui est non substitué ou substitué une ou plusieurs fois par des substituants alkyle en C₁-C₄ ou un groupe oxo,
W représente un atome de O ou de S,
X et Y représentent, indépendamment l'un de l'autre, des atomes d'hydrogène, des atomes d'halogènes, des groupes alkyle en C₁-C₄, alkoxy en C₁-C₄, (alkyl en C₁-C₄)thio, chacun des trois derniers restes cités étant non substitué ou substitué par un ou plusieurs restes pris dans le groupe comprenant des atomes d'halogènes, des groupes alkoxy en C₁-C₃ et (alkyl en C₁-C₄)thio, ou représente des groupes mono- ou di-(alkyl en C₁-C₄)amino, cycloalkyle en C₃-C₆, alcényle en C₃-C₅, alcényloxy en C₃-C₅ ou alcynyloxy en C₃-C₅ et
Z représente des groupes CH ou N.

3. Composés de formule (I) ou leurs sels, **caractérisés en ce que**
R¹ un groupe alkyle en C₁-C₆, qui est non substitué ou substitué par un ou plusieurs restes pris dans le groupe comprenant un atome d'halogène et un groupe alkoxy en C₁-C₄, ou représente 3-oxétanyle, alcényle en C₃-C₄ ou alcynyle en C₃-C₄,
a)
R² et R³ représentent, indépendamment l'un de l'autre, des groupes (alkyl en C₁-C₄)carbonyle, (alkoxy en C₁-C₄)carbonyle, CONR⁷R⁸, CHO, CN, NO₂, benzoyle ou (alkyl en C₁-C₄)sulfonyle ou
b)
CR²R³ représentent ensemble un cycle de 5 à 8 atomes cycliques, qui est carbocylique ou hétérocyclique substitué par un ou deux hétéroatomes pris dans le groupe des atomes de N, O et S et par un ou deux groupes oxo chaque fois en position alpha par rapport a l'atome de carbone en position 1 du groupe CR²R³ et n'est pas substitué en plus ou est substitué en plus par un ou plusieurs restes pris parmi les restes alkyle en C₁-C₄, haloalkyle en C₁-C₄ et halogène, ou
c)
R² représente un groupe NO₂ ou (alkyl en C₁-C₄)carbonyle et
R³ représente un atome d'hydrogène ou un groupe alkyle en C₁-C₄,
R⁴ représente un atome d'hydrogène, un groupe alkyle en C₁-C₄, qui est non substitué ou substitué par un ou plusieurs restes pris dans le groupe comprenant des atomes d'halogènes, des groupes alkoxy en C₁-C₄, (alkyl en C₁-C₄)thio, (alkoxy en C₁-C₄)carbonyle et phényle, ou alcényle en C₃-C₄ ou alcynyle en C₃-C₄,
R⁵ représente un atome d'hydrogène, des groupes alkyle en C₁-C₄, haloalkyle en C₁-C₄, alkoxy en C₁-C₄ ou halogène,
X et Y représentent, indépendamment l'un de l'autre, des groupes alkyle en C₁-C₄, alkoxy en C₁-C₄, chacun des deux derniers restes cités étant non substitué ou substitué par un ou plusieurs atomes d'halogènes, ou représente des restes (alkyl en C₁-C₄)thio, halogène ou mono- ou di-(alkyl en C₁-C₄)amino, et
W représente un atome d'oxygène.

4. Composés ou leurs sels selon une ou plusieurs des revendications 1 à 3, **caractérisés en ce que**
R¹ représente un groupe méthyle ou éthyle,
R² et R³ représentent, indépendamment l'un de l'autre, des groupes (alkoxy en C₁-C₂)carbonyle, CN ou NO₂,
CR²R³ représentent ensemble un cycle de 5 ou 6 atomes cycliques, qui est carbocylique ou hétérocyclique présentant chaque fois un ou deux hétéroatomes pris dans le groupe des atomes de N, O et S et qui est substitué par un ou deux groupes oxo chaque fois en position alpha par rapport à l'atome de carbone en position 1 du groupe CR²R³ et n'est pas substitué en plus ou est substitué en plus par un ou plusieurs restes alkyle en C₁-C₂, et
R⁴ représente un atome d'hydrogène,
R⁵ représente un atome d'hydrogène, des groupes alkyle en C₁-C₄) ou halogène,
X représente des groupes alkyle en C₁-C₂, alkoxy en C₁-C₂, (alkyl en C₁-C₂)thio, halo(alkyl en C₁-C₂)thio ou haloalkoxy en C₁-C₂ et
Y représente des groupes alkyle en C₁-C₂, alkoxy en C₁-C₂, halogène, NHCH₃ ou N(CH₃)₂.

5. Procédé pour la préparation de composés de formule (I) ou de leurs sels tels que définis à la revendication 1 à 4, **caractérisé en ce qu'**on fait réagir
a) un composé de formule (II) 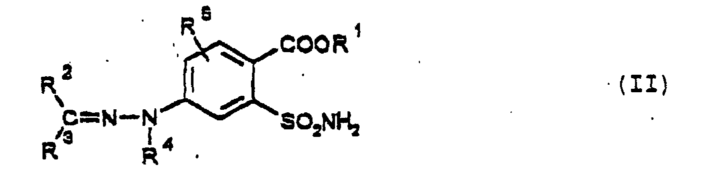 sur un carbamate hétérocyclique de formule (III) 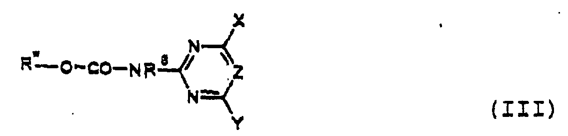 où R* représente un groupe alkyle en C₁-C₄ ou phényle éventuellement substitué, ou
b) un sulfonylcarbamate de formule (IV) 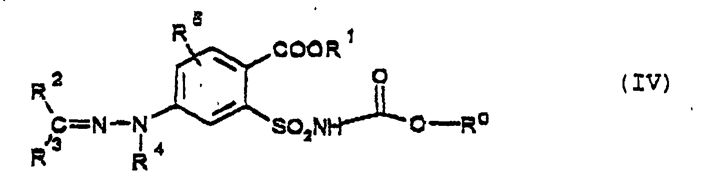 où R⁰ représente un groupe alkyle en C₁-C₄ ou phényle éventuellement substitué, sur un hétérocycle aminé de formule (V) 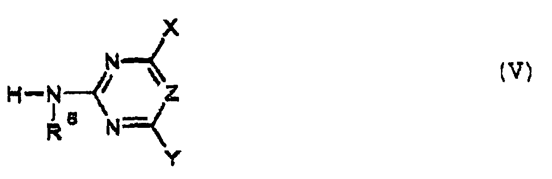 ou
c) un sulfonylisocyanate de formule (VI) 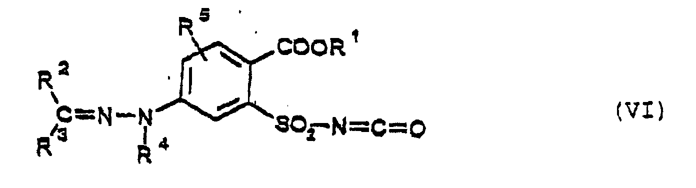 sur un hétérocycle aminé de formule (V) ou
d) un sulfonamide de formule (II) sur un (thio)-isocyanate de formule (VII) 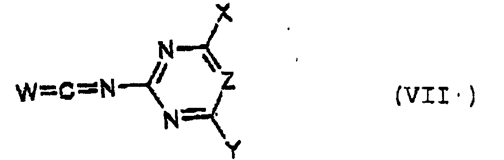 en présence d'une base ou
e) on fait réagir un hétérocycle aminé de formule (V), d'abord catalysé par une base sur un ester d'acide carbonique, et on fait régir le produit intermédiaire obtenu dans une réaction à un pot sur un sulfonamide de formule (II) (voir la variante a),
aux formules (II)-(VII) les restes ou les groupes R¹ à R⁶, W, X, Y et Z sont définis comme à la formule (I) et on obtient, à l'aide de variantes de procédé de a) à c) et e), d'abord des composés de formule (I) où W = O.

6. Herbicides ou agents de croissance végétale **caractérisés en ce qu'**ils contiennent au moins un composé de formule (I) ou leurs sels selon une des revendications 1 à 4 et des adjuvants de formulation usuels en phytoprotection.

7. Procédé pour la lutte contre les dommages sur les plantes ou pour la régulation de la croissance de plantes, **caractérisé en ce qu'**on applique une quantité efficace d'au moins un composé de formule (I) au leurs sels selon l'une des revendications 1 à 4 sur les plantes adventices ou les planter, leurs grains ou la surface, où elles poussent.

8. Utilisation des composés de formule (I) ou leurs sels selon l'une ou plusieurs des revendications 1 à 4, en tant qu'herbicides ou régulateurs de croissance de plantes.

9. Composés de formule (II), (IV) et (VI) définis à la revendication 5.
